# EUROPEAN PATENT SPECIFICATION

(11) **EP 4 023 225 B1**
(45) Date of publication and mention of the grant of the patent: **28.01.2026**
(21) Application number: 20856770.1
(22) Date of filing: 28.08.2020
(51) Int. Cl.: A61K 31/616, A61K 31/495, A61K 45/06, A61P 3/00, A61P 1/16, A61P 3/04, A61P 3/06, A61P 9/10, A61P 27/00, A61P 27/06, A61P 43/00

(54) **COMBINATIONS OF A SALICYLATE AND TRIMETAZIDINE FOR USE IN THE TREATMENT OF METABOLIC DISEASES**
KOMBINIERUNGEN AUS EINEM SALIZYLAT UND TRIMETAZIDIN ZUR BEHANDLUNG VON STOFFWECHSELERKRANKUNGEN
COMBINAISONS D'UN SALICYLATE ET DU TRIMÉTAZIDINE POUR LEUR UTILISATION DANS LE TRAITEMENT DES MALADIES MÉTABOLIQUES

(30) Priority: 30.08.2019 WO PCT/CN2019/103677
(43) Date of publication of application: 06.07.2022
(73) Proprietor: Institute Of Zoology, Chinese Academy Of Sciences, Beijing 100101 (CN)
(72) Inventor: NG, Shyh Chang, Beijing 100101 (CN); MA, Shi Lin, Beijing 100101 (CN)
(74) Representative: CMS Cameron McKenna Nabarro Olswang LLP
(86) International application number: PCT/CN2020/112143
(87) International publication number: WO 2021/037212

(56) References cited:
- CN-A- 101 702 884
- CN-A- 103 384 529
- US-A1- 2011 275 649
- REDDY PRIYA ET AL: "Metabolic syndrome is an inflammatory disorder: A conspiracy between adipose tissue and phagocytes", CLINICA CHIMICA ACTA, vol. 496, 20 June 2019 (2019-06-20), AMSTERDAM, NL, pages 35 - 44, XP093066678, ISSN: 0009-8981, DOI: 10.1016/j.cca.2019.06.019
- MONTI LUCILLA D ET AL: "Metabolic and endothelial effects of trimetazidine on forearm skeletal muscle in patients with type 2 diabetes and ischemic cardiomyopathy", AMERICAN JOURNAL OF PHYSIOLOGY: ENDOCRINOLOGY AND METABOLISM, AMERICAN PHYSIOLOGICAL SOCIETY, BETHESDA, MD, US, vol. 290, no. 1, 20 September 2005 (2005-09-20), pages E54 - E59, XP008092383, ISSN: 0193-1849, DOI: 10.1152/AJPENDO.00083.2005
- WANG, ZHENGUO: "Clinical Effect of Trimetazidine Combined with Aspirin on Treatment for Patients with Coronary Artery Disease and Heart Failure", SHANXI MEDICAL JOURNAL, vol. 48, no. 24, 31 December 2019 (2019-12-31), XP009526482, DOI: 10.3969/j.issn.0253-9926.2019.24.012
- LU YU; LI HAITAO; YANG YOUMING: "Clinical Study of Trimetazidine Combined with Aspirin on Treatment of Unstable Angina: Report of 82 Cases", CHINESE JOURNAL OF TRAUMA AND DISABILITY MEDICINE, vol. 22, no. 10, 31 December 2014 (2014-12-31), pages 143 - 144, XP009526503, ISSN: 1673-6567, DOI: 10.13214/j.cnki.cjotadm.2014.10.127
- OMAR M E ABDEL-SALAM , SIHAM EL-BARAN: "Pharmacological Investigation of Trimetazidine in Models of Inflammation, Pain and Gastric Injury in Rodents", PHARMACOLOGY, vol. 75, no. 3, 9 September 2005 (2005-09-09), pages 122 - 132, XP009086065, ISSN: 0031-7012, DOI: 10.1159/000088211
- GAO YUAN; TANG WEI; LIU CHAO: "Research Progress in the Relationship Between Inflammation and Type 2 Diabetes Mellitus Almost perfect , a space is missing between Type and 2", CHINESE JOURNAL OF CLINICIANS, vol. 4, no. 9, 30 September 2010 (2010-09-30), pages 1635 - 1638, XP009526504, ISSN: 1674-0785, DOI: 10.3877/cma.j.issn.1674-0785.2010.09.064
- RIPUDAMAN S. HUNDAL , KITT F PETERSEN , ADAM B MAYERSON , PRITPAL S RANDHAWA , SILVIO INZUCCHI , STEVEN E SHOELSON , GERARD I SCHO: "Mechanism by which high-dose aspirin improves glucose metabolism in type 2 diabetes.", THE JOURNAL OF CLINICAL INVESTIGATION., vol. 109, no. 10, 15 May 2002 (2002-05-15), pages 1321 - 1326, XP002432772, DOI: 10.1172/JCI200214955

## Description

### Background Art

Metabolic syndrome refers to a disease state in which the metabolism of proteins, fats, carbohydrates and other substances in the human body is disordered. It is not a single disease, but a complex group of metabolic disorders including: abdominal fat accumulation, high blood lipids, high triglyceride, high cholesterol, high blood pressure, high blood sugar level, etc. A central part of metabolic syndrome is obesity and insulin resistance, which mainly includes the components of obesity, especially central obesity. Metabolic syndrome subjects have risk factors for diabetes, cardiovascular and cerebrovascular diseases, fatty liver, polycystic ovary syndrome, and their prevalence of cardiovascular events and risk of death are approximately 2 to 3 times higher than those of non-metabolic syndrome subjects.

There is worldwide prevalence of obesity and type 2 diabetes (see National Diabetes Data Set, American Diabetes, USA: National Institute of Diabetes and Digestive Diseases, National Institutes of Health, 1994; Mokdad et al, Diabetes Care 23(9): 1278-12 S3 (2000); Mokdad et al, JAMA 284(13): 1650-1651 (2000); Mokdad et al, JAMA 286(10): 1195-1200 (2001); Mokdad et al, JAMA 289(1): 76-79 (2003)). In 2000, an estimated 2.9 million people died from diabetes-related causes (Roglic et al., Diabetes Care 28:2130-2135, 2005). The global burden of diabetes is estimated to be double in the next 25 years (King et al, Diabetes Care 21: 1414-1431, 1998; Amos et al, Diabet Med 14 Suppl 5: S1-85, 1997; Wild et al, Diabetes Care 27: 1047-1053, 2004). This occurs in accompany with an increase in obesity. Type 2 diabetes (T2DM) is an increasingly common disease. Due to its high frequency of complications, the life expectancy of the patients is significantly reduced. Type 2 diabetes is currently the most common cause of adult vision loss, renal failure and amputation in the industrialized world due to the microvascular complications associated with diabetes. In addition, the presence of type 2 diabetes is associated with an increased risk of cardiovascular disease.

After the disease has persisted for a long period of time, most subjects with type 2 diabetes eventually become insulin dependent after failing oral therapy, requiring daily injections and multiple daily glucose measurements. The UKPDS (UK Prospective Diabetes Study) has demonstrated that intensive treatment with metformin, sulfonylureas or insulin may result in only limited improvement in glycemic control (difference in HbA1c at ~0.9%). Furthermore, even in subjects within the intensive treatment group, glycemic control deteriorated significantly over time. This is attributed to the deterioration of beta cell function. Importantly, intensive treatment is not associated with a significant reduction in macrovascular complications (that is, cardiovascular events).

Accordingly, there is an unmet medical need for methods, medicaments and pharmaceutical compositions that have good efficacy in glycemic control, in disease-improving properties, and in reducing cardiovascular morbidity and mortality while exhibiting an improved safety profile.

In current studies, obesity-induced inflammation plays a crucial role, especially as the link between elevated blood glucose levels and Cox-2 activation in pancreatic beta cells has been well established. High glucose-induced prostaglandin E2 (PGE2) can lead to a reduction in β-cell mass by inhibiting β-cell proliferation and inducing β-cell apoptosis (Oshima, H. et al., 2006). Indomethacin, a non-selective cyclooxygenase inhibitor, can prevent HFD (high-fat diet)-induced obesity and insulin resistance in C57BL/6J mice (Fjaere E. et al., 2014). The treatment of certain non-steroidal anti-inflammatory drug (NSAID), such as Celecoxib, has been shown to partially restore insulin sensitivity in both translational preclinical models as well as in obese T2DM subjects (Gonzalez-Ortiz et al, 2005). Hyperglycemia activates Cox-2 in pancreatic beta cells and leads to beta cell dysfunction. The treatment with NS-398, a selective Cox-2 inhibitor, can reverse β-cell dysfunction by reducing PGE2-mediated β-cell apoptosis (Tian, V.F. et al., 2014). Inflammation due to obesity may lead to nonalcoholic steatosis, a pathological hallmark of insulin resistance. Nonalcoholic steatohepatitis (NASH) is a coexisting condition with T2DM. Celecoxib can reverse steatohepatitis and inflammation in the HFD-induced Wistar rat NASH model (Chen, J. et al., 2011). Overexpression of NAG-1/GDF-15 (NSAID-activated gene-1) has been shown to improve glycemic parameters and prevent the development of obesity by increasing thermogenesis, lipolysis and oxidative metabolism in obese C57BL/6J mice (Chrysovergis, K. et al., 2014). Activation of an inducible form of Cox-2 may play a critical role in the initiation of cellular dysfunction, including: adipocyte dysfunction, pancreatic beta cell dysfunction, and macrophage dysfunction. Cell dysfunction contributes to the development of insulin resistance and systemic glucose intolerance. Cox-2 deletion in C57BL/6J obese mice reduced blood glucose levels (Fujta et al., 2007). More importantly, the selective Cox-2 inhibitor celecoxib can slightly reduce the HbA1c level, improve glucose tolerance and increase the insulin level in the same translational preclinical model (Fujita, H. et al., 2007). Based on preclinical and clinical data, treating the underlying inflammatory components of the complex pathophysiology of type 2 diabetes with anti-inflammatory therapy can be one of the strategies for partial remission and management of type 2 diabetes.

In addition, a number of publications have supported the importance of adipocytes and inflammation in the development of insulin resistance. For example, JNK-1 deficiency in adipocytes suppresses high-fat diet-induced insulin resistance in the liver due to JNK dependence (Sabio, C. et al., 2008); adipocyte-specific Glut4 deletion or MCP-1 overexpression leads to systemic insulin resistance (Qi, L. et al., 2009); TNF-α deficiency improves insulin sensitivity in diet-induced obesity and in the Lep^{ob/ob} model of obesity (Hotamisiligil, GS et al., 1995); elevated IL-1β, IL-6 and CRP predict the development of T2DM (Visser, M. et al., 1999); TLR4 knockout mice can be protected from inflammation and insulin resistance (Shi, H. et al., 2006). Current hypoglycemic drugs do not involve inflammatory inhibitors at all, and thus lack sufficient efficacy and lack sufficient overall clinical benefit. This is due to their inability to inhibit the pro-inflammatory components of the complex pathophysiology that initiate and maintain systemic insulin resistance.

Inflammation is not only an important part of the pathophysiology of type 2 diabetes but also an important part of clinically relevant comorbidities. What's more, pro-inflammatory signals help initiate and maintain complications associated with type 2 diabetes such as diabetic retinopathy, skin ulcers, coronary heart disease (CHD), stroke, fatty liver, polycystic ovary syndrome, chronic kidney disease (CKD), diabetic peripheral neuropathy, diabetic vascular diseases, etc. Pro-inflammatory signals can determine the severity and duration of diabetes-related complications. There is a direct association between elevated pro-inflammatory biomarkers and impaired glucose metabolism. Therefore, to achieve effective clinical management, it is important to jointly regulate inflammation and metabolism, and uniformly treat metabolic diseases including type 2 diabetes, which may include all metabolic syndrome disorders of complex pathophysiology with a strong pro-inflammatory component, rather than treating each metabolic disease as a separate individual disease. At present, NSAID drugs alone cannot comprehensively inhibit many inflammatory factors and inflammatory pathways, and hypoglycemic drugs alone cannot fully restore glucose and lipid metabolism. For example, in order to obtain an adequate therapeutic effect in subjects with type 2 diabetes, fatty liver, polycystic ovary syndrome or obesity, it would be necessary to treat with a combination of drugs that jointly modulate inflammation and metabolism. This not only corrects impaired blood glucose and lipid homeostasis, but also alleviates clinically relevant metabolic comorbidities. More importantly, this can reduce the severity of diabetes-related complications. Therefore, subjects with metabolic syndrome should not be treated with only one drug, but should be treated with a combination of anti-inflammatory drugs and anti-metabolic disease drugs. WO2008111956A2 relates to methods, compositions and kits for treating hyperglycemia and related disorders, such as type 2 diabetes mellitus, impaired glucose tolerance, diabetic retinopathy, diabetic nephropathy and diabetic neuropathy, and to methods, compositions and kits for treating erectile dysfunction. The methods comprise administering an inhibitor of fatty acid oxidation to a subject in need thereof.

### Summary of the Invention

The invention is as defined in the claims. The following aspects are useful for understanding the invention but are not part of the claimed invention in their entirety. The reference to methods of treatment by therapy or surgery or *in vivo* diagnosis methods in the examples of this description are to be interpreted as references to compounds, pharmaceutical compositions and medicaments of the present invention for use in those methods.

Any reference to therapeutic agent A herein is to salicylate. Any reference to therapeutic agent B herein is to trimetazidine.

The object of the present disclosure is to overcome the deficiencies in the existing technologies, that is, the existing drugs on the market cannot effectively treat or prevent the metabolic syndrome caused by obesity, type 2 diabetes and insulin resistance. Thus, the present disclosure provides a pharmaceutical composition that not only corrects impaired glucose homeostasis, but also alleviates clinically relevant comorbidities, and more importantly, reduces the severity of diabetes-related complications.

In order to achieve the object mentioned above, in one aspect, the present invention provides a pharmaceutical composition, which comprises a therapeutic agent A or a pharmaceutically acceptable salt thereof; a therapeutic agent B or a pharmaceutically acceptable salt thereof; and at least one pharmaceutically acceptable excipient, where the therapeutic agent A is a non-steroidal anti-inflammatory drug, and the therapeutic agent B is a fatty acid oxidation inhibitor.

In one embodiment, in the pharmaceutical composition, the therapeutic agent A and the therapeutic agent B are contained in a single dosage form.

In another embodiment, in the pharmaceutical composition, the therapeutic agent A and the therapeutic agent B are present in separate dosage forms.

The therapeutic agent A is salicylate, or disclosed herein ibuprofen, indomethacin, flurbiprofen, phenoxyibuprofen, naproxen, nabumetone, piroxicam, phenylbutazone, diclofenac, fenprofen, ketoprofen, ketorolac, tetraclofenamic acid, sulindac, and tolmetin.

The therapeutic agent B is trimetazidine, or disclosed herein etomoxir, aminocarnitine or a phosphonooxy derivative of carnitine.

In some embodiments, the present invention provides a pharmaceutical composition, which comprises a therapeutic agent A or a pharmaceutically acceptable salt thereof; a therapeutic agent B or a pharmaceutically acceptable salt thereof; and at least one pharmaceutically acceptable excipient, where the therapeutic agent A is a salicylate, and the therapeutic agent B is trimetazidine.

In some embodiments, the present invention provides a pharmaceutical composition, which comprises a therapeutic agent A or a pharmaceutically acceptable salt thereof; a therapeutic agent B or a pharmaceutically acceptable salt thereof; and at least one pharmaceutically acceptable excipient, where the therapeutic agent A is aspirin, and the therapeutic agent B is trimetazidine.

In some embodiments, the therapeutic agent A (a salicylate, such as aspirin) and the therapeutic agent B (trimetazidine) are in a weight ratio of 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the pharmaceutical dosage form thereof is an oral dosage form. In some embodiments, the pharmaceutical dosage form is an injectable dosage form.

In another aspect, the present disclosure further provides the use (or method) of the therapeutic agent A and the therapeutic agent B in the preparation of a medicament for treating type 1 diabetes, type 2 diabetes, impaired glucose tolerance, impaired fasting glucose, hyperglycemia, postprandial hyperglycemia, overweight, obesity and metabolic syndrome; or improving glycemic control and/or reduce fasting plasma glucose, postprandial plasma glucose and/or HbA1c; or slowing, delaying or reversing the progression from impaired glucose tolerance, impaired fasting glucose, insulin resistance and/or metabolic syndrome to type 2 diabetes; or treating diabetic complications such as cataracts, as well as microvascular and macrovascular diseases such as nephropathy, retinopathy, neuropathy, tissue ischemia, arteriosclerosis, myocardial infarction, stroke and peripheral arterial occlusive disease; or losing weight or promoting weight loss; or treating pancreatic β-cell degeneration and/or decreased pancreatic β-cell function, and/or restoring pancreatic β-cell function, and/or restoring pancreatic insulin secretion; or treating a disease or condition that causes abnormal buildup of fat in the liver; or maintaining and/or improving insulin sensitivity and/or treating hyperinsulinemia and/or insulin resistance; or treating atherosclerosis and complications of atherosclerosis; or treating glaucoma and glaucoma complications; or treating dyslipidemia/hyperlipidemia and complications of dyslipidemia/hyperlipidemia, or treating reproductive-related metabolic diseases in a subject in need thereof, or a treatment method thereof, in which the therapeutic agent A is a non-steroidal anti-inflammatory drug, and the therapeutic agent B is a fatty acid oxidation inhibitor.

In one embodiment, the subject is an individual who is diagnosed with one or more conditions selected from overweight, obesity, visceral obesity, and abdominal obesity.

In another embodiment, the subject is an individual who is diagnosed with one or more of the following conditions:
(a) a fasting blood glucose or serum glucose concentration is greater than 110 mg/dL, especially greater than 125 mg/dL;
(b) a postprandial plasma glucose concentration is equal to or greater than 140 mg/dL; and
(c) an HbA1c value is equal to or greater than 6.5%, especially equal to or greater than 8.0%.

In another embodiment, the subject is an individual with one or more of the following conditions:
(a) obesity, visceral obesity and/or abdominal obesity;
(b) a triglyceride blood concentration ≥ 150mg/dL;
(c) an HDL-cholesterol blood level < 40 mg/dL in a female subject, or an HDL-cholesterol blood level < 50mg/dL in a male subject;
(d) systolic blood pressure ≥ 130 mmHg, diastolic blood pressure ≥ 85 mmHg;
(e) a fasting blood glucose level ≥ 110 mg/dL; and
(f) an LDL-cholesterol blood level ≥ 130 mg/dL.

In another embodiment, the subject is an individual who is contraindicated in metformin monotherapy and/or intolerant to therapeutic doses of metformin.

In another embodiment, the subject is an individual with insufficient glycemic control after the treatment with one or more antidiabetic drugs selected from the group consisting of:
(a) a biguanide;
(b) a sulfonylurea;
(c) a meglitinide;
(d) a thiazolidinedione;
(e) an α-glucosidase inhibitor;
(f) insulin and an insulin analog;
(g) a dipeptidyl peptidase IV inhibitor;
(h) an SGLT2 inhibitor;
(i) a PPARα modulator;
(j) a glucose-dependent insulinotropic polypeptide agonist;
(k) a β-3 agonists;
(l) GLP1 and a GLP1 analog;
(m) a PPARγ modulator; and
(n) an HMG-CoA reductase inhibitor.

The pharmaceutical composition according to the present invention shows very good efficacy in glycemic control, especially in the reduction of fasting plasma glucose, postprandial plasma glucose and/or glycosylated hemoglobin (HbA1c); at the same time, it can effectively treat or prevent metabolic syndrome diseases caused by obesity, non-alcoholic fatty liver disease, polycystic ovary syndrome, type 2 diabetes and insulin resistance.

The therapeutic agent A is selected from a salicylate and the therapeutic agent B is selected from trimetazidine.

In some embodiments, the therapeutic agent A is selected from aspirin and the therapeutic agent B is selected from trimetazidine.

In some embodiments, the therapeutic agent A (a salicylate, such as aspirin) and the therapeutic agent B (trimetazidine) are in a weight ratio of 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the therapeutic agent A and the therapeutic agent B are administered simultaneously. In some embodiments, the therapeutic agent A and the therapeutic agent B are contained in a single dosage form. In some embodiments, the single pharmaceutical dosage form is an oral dosage form.

In some embodiments, the therapeutic agent A and the therapeutic agent B are administered separately. In some embodiments, the therapeutic agent A is administered before the therapeutic agent B. In some embodiments, the therapeutic agent A is administered after the therapeutic agent B. In some embodiments, therapeutic agent A and therapeutic agent B are administered orally, respectively. In some embodiments, therapeutic agent A and therapeutic agent B are administered separately by injection.

Other features and advantages of the present invention will be described in detail in the following description of specific embodiments.

### Brief Description of the Drawings

The accompanying drawings are provided to facilitate further understanding of the present invention and constitute a part of this specification; the drawings are used to explain the present invention along with the following specific embodiments, but do not constitute a limitation of the present invention. In the drawings:
Fig. 1 shows the results of weight changes in diet-induced obesity (DIO) mice after 10 days of treating with the pharmaceutical therapeutic agents A+B in an experimental group using a pharmaceutical composition according to an embodiment of the present invention, as compared with the control group(s).
Fig. 2 shows the results of fat reduction in DIO mice induced by treating with the pharmaceutical therapeutic agents A+B in an experimental group using a pharmaceutical composition according to an embodiment of the present invention, as compared with the control group(s).
Fig. 3 shows the results of the morphology of adipocytes in the gonadal fat pad in an experimental group treated with the pharmaceutical composition according to an embodiment of the present invention, as compared with a DIO control group(s).
Fig. 4 shows the results of fatty liver and liver cell morphology in an experimental group treated with the pharmaceutical composition according to an embodiment of the present invention, as compared with a DIO control group(s).
Fig. 5 shows the results of cardiac muscle cell morphology in an experimental group treated with the pharmaceutical composition according to an embodiment of the present invention, as compared with a DIO control group(s).
Fig. 6 shows the results of skeletal muscle cell morphology in an experimental group treated with the pharmaceutical composition according to an embodiment of the present invention, as compared with a DIO control group(s).
Fig. 7 shows the results of renal toxicity indexes serum creatinine (CREA) and blood urea nitrogen (BUN) in an experimental group treated with the pharmaceutical composition according to an embodiment of the present invention, as compared with a DIO control group(s).
Fig. 8 shows the results of weight changes in diabetic DIO mice after 4 weeks of pharmaceutical therapeutic agents A+B treatment in an experimental group treated with the pharmaceutical composition according to an embodiment of the present invention, as compared with the control group(s).
Fig. 9 shows the comparison of the total amount of serum cholesterol, an indicator associated with hyperlipidemia, in each group of DIO mice.
Fig. 10 shows the comparison of serum LDL-cholesterol, a marker associated with hyperlipidemia, in each group of DIO mice.
Fig. 11 shows the comparison of serum alanine aminotransferase (ALT), an indicator associated with fatty liver, in each group of DIO mice.
Fig. 12 shows the comparison of serum albumin/globulin ratio, an indicator associated with nitrogen metabolism function, in each group of DIO mice.
Fig. 13 shows the comparison of serum total protein, an indicator associated with nitrogen metabolism function, in each group of DIO mice.
Fig. 14 shows the results of glucose tolerance in diabetic DIO mice.
Fig. 15 shows the results of insulin sensitivity in diabetic DIO mice.
Fig. 16 shows the comparison of body weights in DIO mice following administration of various therapeutic agent combinations.
Fig. 17 shows the comparison of fasting blood glucose levels in DIO mice following administration of various therapeutic agent combinations.
Fig. 18 shows the results of body weight change in common wild-type mice induced by the pharmaceutical therapeutic agents A+B in an experimental group treated with the pharmaceutical composition according to an embodiment of the present invention, as compared with the control group(s).
Fig. 19 shows the results of fat reduction in common wild-type mice induced by the pharmaceutical therapeutic agents A+B in an experimental group treated with the pharmaceutical composition according to an embodiment of the present invention, as compared with the control group(s).
Fig. 20 shows the results of body weight change in polycystic ovary syndrome rats induced by the pharmaceutical therapeutic agents A+B in an experimental group treated with the pharmaceutical composition according to an embodiment of the present invention, as compared with the control group(s).
Fig. 21 shows the comparison of fasting blood glucose levels in each group of polycystic ovary syndrome rats.
Fig. 22 shows the comparison of serum aspartate aminotransferase (AST) and total cholesterol, indicators associated with fatty liver, in each group of polycystic ovary syndrome rats.
Fig. 23 shows the comparison of serum total protein, an indicator associated with liver nitrogen metabolism function, in each group of polycystic ovary syndrome rats.
Fig. 24 shows the comparison of serum creatinine, an indicator associated with renal function, in each group of polycystic ovary syndrome rats.
Fig. 25 shows the comparison of serum lactate dehydrogenase (LDH), creatine kinase (CK), cardiac creatine kinase isoenzyme MB (CKMB), α-hydroxybutyric acid dehydrogenase (HBDH), indicator associated with heart diseases, in each group of polycystic ovary syndrome rats.
Fig. 26 shows the results of the insulin sensitivity in polycystic ovary syndrome rats.
Fig. 27 shows the results of the estrous cycle in polycystic ovary syndrome rats.
Fig. 28 shows the results of the serum hormone ELISA in polycystic ovary syndrome rats.
Fig. 29 shows the results of Western blot analysis of skeletal muscle proteins in polycystic ovary syndrome rats.
Fig. 30 shows the results of left ventricular wall thickness analysis in polycystic ovary syndrome rats.
Fig. 31 shows the results of myocardial fibrosis analysis in polycystic ovary syndrome rats.
Fig. 32 shows the results of routine blood analysis in polycystic ovary syndrome rats.
Fig. 33 shows the results of pharmaceutical therapeutic agents A+B on body weight change in NASH mice.
Fig. 34 shows the results of the food intake test in NASH mice.
Fig. 35 shows the results of the liver weight test in NASH mice.
Fig. 36 shows the results of the liver section analysis in NASH mice.
Fig. 37 shows the results of the heart slice analysis in NASH mice.
Fig. 38 shows the comparison of two indicators associated with liver injury in each group of mice.
Fig. 39 shows the comparison of indicators associated with hyperlipidemia in each group of mice.
Fig. 40 shows the comparison of the indicators associated with organ damage in each group of mice.
Fig. 41 shows the results of fasting blood glucose in each group of mice.
Fig. 42 shows the results of glucose tolerance in NASH mice.
Fig. 43 shows the results of insulin sensitivity in NASH mice.
Fig. 44 shows the results of Western blot analysis of skeletal muscle proteins in NASH mice.
Fig. 45 shows the results of transcriptomic sequencing analysis in NASH mice.
Fig. 46 shows the results of protein Western blot analysis of primary human skeletal muscle cells in vitro 24 hours post administration.
Fig. 47 shows the results of protein Western blot analysis of primary human skeletal muscle cells induced insulin resistance in vitro 7 days post administration.
Fig. 48 shows the results of skeletal muscle transcriptomic sequencing analysis induced by high-fat and high-sugar diet in PCOS polycystic ovary syndrome rats.
Fig. 49 shows insulin ELISA results and insulin resistance indicator HOMA-IR results in serum samples in NASH mice.
Fig. 50 shows adiponectin ELISA results in serum samples in NASH mice.
Fig. 51 shows the comparative results of liquid mass spectrometry analysis in serum samples of mice in control group 1 and control group 3.

### Description of embodiments

Specific embodiments of the present disclosure will be described in detail below.

The endpoints of ranges and any values disclosed herein are not limited to the precise ranges or values. These ranges or values should be understood to encompass values close to those ranges or values. For numerical ranges, endpoints of each range, endpoints of each range and individual point values, and individual point values can all be combined with each other to obtain one or more new numerical ranges, and these numerical ranges should be considered as specifically disclosed herein.

The present invention is largely based on the unexpected results obtained by the inventors in the combined use of a non-steroidal anti-inflammatory drug (therapeutic agent A) and a fatty acid oxidation inhibitor (therapeutic agent B). The inventors have surprisingly found that when a subject with diabetes, cardiovascular and cerebrovascular disease, fatty liver, or polycystic ovary syndrome is administered both the therapeutic agent A and the therapeutic agent B, multiple indicators of the subject obtained are improved. Moreover, the drug combination can also achieve the effect of reversing a variety of symptoms. The inventors also surprisingly found that the mechanism of action of the drug combination is not limited to regulating inflammatory responses and metabolism. Daily administration of the drug combination can also specifically make the p38 and AMPK signaling pathways and related metabolic changes cycle again and again so as to form a cycle of excitatory effects, which functions, just like imitating exercise. After short-term administration (within 60 minutes), the pharmaceutical therapeutic agents A+B provided by the present disclosure can jointly regulate p38 and AMPK signaling pathway mechanism model through fatty acid oxidation (FAO), fatty acid metabolites (acyl-metabolites), and adenosine triphosphate (ATP), so as to make the p38 and AMPK signaling pathways increase simultaneously, thereby promoting catabolism such as fat hydrolysis and fatty acid oxidation. In addition, after long-term administration (3 to 24 hours), the pharmaceutical therapeutic agent A+B provided by the present disclosure can jointly regulate p38 and AMPK signaling pathway mechanism model through inflammatory cytokines (Inf cytokines), fatty acid oxidation (FAO), mitochondrial reactive oxygen species (mtROS), glycolysis (glycolysis), resulting in simultaneous plummeting of p38 and AMPK signaling pathways, thereby promoting anabolism and muscle repair. The present patent application thus provides a novel and effective pharmaceutical composition on the one hand, and a method of treatment using the pharmaceutical composition on the other hand.

### Pharmaceutical composition

In one aspect, the present disclosure provides a pharmaceutical composition comprising a therapeutic agent A or a pharmaceutically acceptable salt thereof; a therapeutic agent B or a pharmaceutically acceptable salt thereof; and at least one pharmaceutically acceptable excipient, in which the therapeutic agent A is a non-steroidal anti-inflammatory drug, and the therapeutic agent B is a fatty acid oxidation inhibitor.

The term "pharmaceutically acceptable" refers to those compounds, materials, compositions and/or dosage forms within the scope of sound medical judgment, suitable for use in contact with human and animal tissues without undue toxicity, irritation, allergy or other problems or complications, and with reasonable benefit/risk ratios. The therapeutic Agent A and therapeutic Agent B herein can form stable pharmaceutically acceptable acid or base salts, and in such case it may be appropriate to administer the compound as a salt. Examples of the acid salts include: acetate, adipate, ascorbate, benzoate, besylate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-isethionate, heptanoate, caproate, hydrochloride, hydrobromide, hydroiodide, hydroxymaleate, lactate, malate, maleate, mesylate, meglumine, 2-naphthalenesulfonate acid, nitrate, oxalate, pamoate, persulfate, phenyl acetate, phosphate, diphosphate, picrate, pivalate, propionate, quinate, salicylate, stearate, succinate, sulfamate, sulfamate, sulfate, tartrate, tosylate (p-toluenesulfonate), trifluoroacetate and undecyl acid ester. Examples of base salts include: ammonium salts; alkali metal salts such as sodium, lithium and potassium salts; alkaline earth metal salts such as aluminum, calcium and magnesium salts; salts with organic bases such as dicyclohexylamine and N-methyl-D-glucosamine, as well as salts with amino acids such as arginine, lysine, ornithine, and the like.

In one embodiment, the therapeutic agent A and therapeutic agent B in the pharmaceutical composition may be present in therapeutically effective amounts. As used herein, the term "therapeutically effective amount" refers to an amount of a compound or composition sufficient to significantly and positively alter the symptom and/or condition being treated (e.g., to provide a positive clinical response). The effective amount of the active ingredient used in the pharmaceutical composition may vary based on the particular condition being treated, the severity of the condition, the duration of treatment, the nature of the concurrent therapy, the particular active ingredient employed, the particular pharmaceutically acceptable excipient employed, and similar factors within the knowledge and expertise of the attending physician.

As used herein, the term "treatment" refers to a method for obtaining beneficial or desired clinical results. The term "treating" means inhibiting, preventing or arresting the development or progression in pathology (disease, disorder or condition) and/or causing a reduction, remission or regression in pathology. A person of ordinary skill in the art will appreciate that various methods and assays can be used to assess the development of pathology, and similarly, various methods and assays can be used to assess the reduction, remission or regression in pathology.

As used herein, the term "prevention" refers to preventing a disease, disorder, or condition from developing in a subject who may be at risk for the disease but has not been diagnosed with the disease. Prevention (and doses effective for prevention) can be demonstrated in population studies. For example, an amount effective to prevent a given disease or medical condition is an amount effective to reduce the incidence in a treated population relative to an untreated control population.

As used herein, the term "subject" may include mammals, preferably humans of any age with pathological features. Preferably, the term may also include individuals at risk of developing pathological features.

### Therapeutic A and therapeutic B

According to the present disclosure, the therapeutic agent A is a non-steroidal anti-inflammatory drug. As used herein, the term "non-steroidal anti-inflammatory drugs (NSAIDs)" is a class of anti-inflammatory drugs that do not contain steroid structures. It can be mainly divided into three categories: acetylsalicylate, including aspirin (acetylsalicylic acid); non-acetylsalicylate, including magnesium salicylate, sodium salicylate, magnesium choline salicylate, diflunisal, and salsalate; and nonsalicylate, including ibuprofen, indomethacin, flurbiprofen, phenoxyibuprofen, naproxen, nabumetone, piroxicam, phenylbutazone, diclofenac, fenprofen, ketoprofen, ketorolac, tetraclofenamic acid, sulindac, tolmetin, and the like. In addition, the therapeutic agent A in the present disclosure can be selected from any of these non-steroidal anti-inflammatory drugs. The therapeutic agent A for use in the invention is a salicylate, or disclosed herein ibuprofen, indomethacin, flurbiprofen, phenoxyibuprofen, naproxen, nabumetone, piroxicam, phenylbutazone, diclofenac, fenprofen, ketoprofen, ketorolac, tetraclofenamic acid, sulindac, and tolmetin. The therapeutic agent A for use in the invention is a salicylate such as aspirin or magnesium salicylate, and the like.

According to the present disclosure, the therapeutic agent B is a fatty acid oxidation inhibitor including, but not limited to, etomoxir, meldonium, oxfenicine, perhexiline, ranolazine, substituted piperazines, trimetazidine and carnitine derivatives. The therapeutic agent B for use in the invention is trimetazidine, or disclosed herein aminocarnitines (for example, as described in WO85/04396), phosphinyloxy derivatives of carnitine (for example, as described in EP0574355B1), or complex products of carnitine with other compounds (for example, as described in JP5127093B2/US6369073B1). The therapeutic agent B for use in the invention is trimetazidine.

According to the present invention, in the pharmaceutical composition of the present invention, the combination of the therapeutic agent A and the therapeutic agent B is not particularly limited. That is, in the pharmaceutical composition of the present invention, the therapeutic agent A and the therapeutic agent B may be contained in a single dosage form, or may also be present in separate dosage forms. Thus, upon subsequent use of the pharmaceutical composition of the present invention, the therapeutic agent A and the therapeutic agent B can be administered to the subject simultaneously, separately or sequentially. For example, the therapeutic agent A and the therapeutic agent B can be administered to a subject simultaneously (such as in the same dosage form). The therapeutic agent A can be administered to the subject followed by the administration of the therapeutic agent B to the subject immediately, or therapeutic agent B can be administered to the subject within 1 hour, 2 hours, 3 hours, 4 hours, 5 hours, 6 hours, 7 hours, or 8 hours after administration of the therapeutic agent A to the subject. In some embodiments, the therapeutic agent B can be administered to the subject immediately followed by administering the therapeutic agent A to the subject. Alternatively, the therapeutic agent A can be administered to the subject within 1 hour, 2 hours, 3 hours, 4 hours, 5 hours, 6 hours, 7 hours, or 8 hours after administration of the therapeutic agent B to the subject.

### Administration methods

The pharmaceutical compositions of the present invention is suitable for oral administration (for example, such as tablets, lozenges, hard or soft capsules, aqueous or oily suspensions, emulsions, dispersible powders or granules, syrups, or elixirs), topical administration (for example, such as a cream, ointment, gel, or aqueous or oily solution or suspension), inhalation administration (for example, such as a fine powder or liquid aerosol), insufflation administration (for example, such as a fine powder), or parenteral administration (for example, such as sterile aqueous or oily solutions for intravenous, subcutaneous, intramuscular or intramuscular administration, or suppositories for rectal administration). In a preferred embodiment, the pharmaceutical composition of the present invention is administered orally.

The pharmaceutical compositions of the present invention can be obtained by conventional procedures using conventional pharmaceutical excipients well known in the art. Suitable pharmaceutically acceptable excipients for tablet formulation include, for example, inert diluents, such as lactose, sodium carbonate, calcium phosphate or calcium carbonate; granulating and disintegrating agents, such as corn starch or alginic acid; binding agents such as starch; lubricants, such as magnesium stearate, stearic acid, or talc; preservatives, such as ethyl or propylparaben; and antioxidants, such as ascorbic acid. Tablet formulations may be uncoated or coated. Coatings are intended to either modify the disintegration in the gastrointestinal tract and subsequent absorption of the active ingredient, or to improve the stability and/or appearance. In both cases, conventional coating agents and methods well known in the art can be used.

The pharmaceutical composition of the present invention is not strictly limited in terms of dosage and frequency of administration. They may vary depending on many factors, such as age, weight, general health, diet, sex, drug to be administered, route (or method) of administration, and severity of the condition being treated, as well as the judgment of the attending physician. In general, the pharmaceutical compositions of the present invention may be administered one or more times per day, such as once a day, two times a day, three times a day or more, and can also be administered every two days, every three days, once a week, or at other frequencies. In terms of daily dosage, the amount of the therapeutic agent A may be administered from 0.1 mg to 5000 mg per day, or preferably from 10 mg to 3000 mg, more preferably from 80 mg to 2000 mg, or even more preferably from 500 mg to 1500 mg. The amount of the therapeutic agent B may be administered 0.1 mg to 1000 mg per day, or preferably 10 mg to 1000 mg, more preferably 100 mg to 500 mg, or even more preferably 35 mg to 300 mg. Therefore, the application of the above-mentioned dosage can be reasonably achieved according to the above-mentioned different application frequencies and methods. For example, 5 mg to 500 mg of the therapeutic agent A is administered once a day, or 10 mg to 250 mg of the therapeutic agent A is administered twice a day, and the like.

The pharmaceutical composition comprises a salicylate (such as 100, 200, 300, 400, 500, or 600 mg of salicylate), and trimetazidine (such as 10, 20, 30, 40, 50, 60, 70, 80, 90, or 100 mg of trimetazidine). In some embodiments, the pharmaceutical composition comprises aspirin (such as 100, 200, 300, 400, 500, or 600 mg of aspirin), and trimetazidine (such as 10, 20, 30, 40, 50, 60, 70, 80, 90, or 100 mg of trimetazidine). In some embodiments, the weight ratio of salicylate (such as aspirin) to trimetazidine is from 1:1 to 10:1, including 2:1, 3:1, 4:1, 5:1, 6:1 1, 7:1, 8:1, 9:1, or 10:1.

### Use (or method)

The present invention provides the use of the therapeutic agent A and the therapeutic agent B in the preparation of a medicament for use as defined in the claims.
In another aspect, the present disclosure further provides the use (or method, said methods do not form part of the present invention) of the therapeutic agent A and the therapeutic agent B in the preparation of a medicament for treating type 2 diabetes, impaired glucose tolerance, impaired fasting glucose, hyperglycemia, postprandial hyperglycemia, type 1 diabetes, overweight, obesity and metabolic syndrome; or improving glycemic control and/or reduce fasting plasma glucose, postprandial plasma glucose and/or HbA1c A1c; or slowing, delaying or reversing the progression from impaired glucose tolerance, impaired fasting glucose, insulin resistance and/or metabolic syndrome to type 2 diabetes; or treating diabetic complications such as cataracts, as well as microvascular and macrovascular diseases such as nephropathy, retinopathy, neuropathy, tissue ischemia, arteriosclerosis, myocardial infarction, stroke and peripheral arterial occlusive disease; or losing weight or promoting weight loss; or treating pancreatic beta cell degeneration and/or decreased pancreatic beta cell function, and/or restoring pancreatic beta cell function, and/or restoring pancreatic insulin secretion; or treating a disease or condition that causes abnormal buildup of fat in the liver; or maintaining and/or improving insulin sensitivity and/or treating hyperinsulinemia and/or insulin resistance; or treating atherosclerosis and complications of atherosclerosis; or treating glaucoma and glaucoma complications; or treating dyslipidemia/hyperlipidemia and complications of dyslipidemia/hyperlipidemia in a subject in need thereof, in which the therapeutic agent A is a non-steroidal anti-inflammatory drug, and the therapeutic agent B is a fatty acid oxidation inhibitor.

In some embodiments, the present disclosure provides the use (or method, said methods do not form part of the present invention) of the therapeutic agent A and the therapeutic agent B in the preparation of a medicament for treating type 2 diabetes, impaired glucose tolerance, impaired fasting glucose, type 1 diabetes, obesity, steatohepatitis, atherosclerosis, glaucoma, dyslipidemia/hyperlipidemia, and hyperglycemia in a subject in need thereof, in which the therapeutic agent A is a non-steroidal anti-inflammatory drug, and the therapeutic agent B is a fatty acid oxidation inhibitor. In some embodiments, the present disclosure provides a use (or method, said methods do not form part of the present invention) of treating one or more of the disorders including type 2 diabetes, impaired glucose tolerance, impaired fasting glucose, type 1 diabetes, obesity, steatohepatitis, atherosclerosis, glaucoma, dyslipidemia/hyperlipidemia, and hyperglycemia in a subject in need thereof, which comprises administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for the treatment of type 1 diabetes or type 2 diabetes in a subject in need thereof, where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) of treating type 1 diabetes or type 2 diabetes in a subject in need thereof, comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for the treatment of a disease or disorder resulting in abnormal accumulation of hepatic fat in a subject in need thereof, where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) of treating a disease or condition resulting in abnormal accumulation of hepatic fat in a subject in need thereof, comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a reproductive-related metabolic disease in a subject in need thereof, where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) of treating a reproductive-related metabolic disease in a subject in need thereof, comprising administering the therapeutic agent A and the therapeutic agent B to an afflicted individual, where the therapeutic agent A is a non-steroidal anti-inflammatory drug, and the therapeutic agent B is a fatty acid oxidation inhibitor. The reproductive-related metabolic disease herein includes, but is not limited to, polycystic ovary syndrome (PCOS), gestational diabetes, preeclampsia, recurrent spontaneous abortion, fetal growth restriction, ovarian insufficiency, premature ovarian failure, and male infertility. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present invention provides the use (or method, said methods do not form part of the present invention) of the therapeutic agent A and the therapeutic agent B in the preparation of a medicament for the treatment of polycystic ovary syndrome (PCOS) in an afflicted individual in need thereof, wherein, the therapeutic agent A is a non-steroidal anti-inflammatory drug according to the claims, and the therapeutic agent B is a fatty acid oxidation inhibitor according to the claims. In some embodiments, the present disclosure provides a method of treating polycystic ovary syndrome (PCOS), comprising administering the therapeutic agent A and the therapeutic agent B to an afflicted individual, where the therapeutic agent A is a non-steroidal anti-inflammatory drug according to the claims, and the therapeutic agent B is a fatty acid oxidation inhibitor according to the claims. Said methods do not form part of the present invention. The therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A ( a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for the restoration of skeletal muscle insulin sensitivity and protein anabolism (or alleviation of muscle inflammation, insulin resistance, sarcopenia and/or metabolic syndrome) in an afflicted individual in need thereof (such as a patient with polycystic ovary syndrome), where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) for the restoration of skeletal muscle insulin sensitivity and protein anabolism (or alleviation of muscle inflammation, insulin resistance, sarcopenia and/or metabolic syndrome) in an afflicted individual in need thereof (such as a patient with polycystic ovary syndrome), comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for ameliorating (including reversing) cardiovascular diseases such as myocardial fibrosis, ventricular hypertrophy, heart failure, etc., in an afflicted individual in need thereof (such as a patient with polycystic ovary syndrome or NASH), where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) for ameliorating (including reversing) cardiovascular diseases such as myocardial fibrosis, ventricular hypertrophy, heart failure, etc., in an afflicted individual in need thereof (such as a patient with polycystic ovary syndrome or NASH), comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for the reversal of insulin resistance, sarcopenia and metabolic syndrome in an afflicted individual in need thereof (such as a patient with polycystic ovary syndrome or NASH), where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) for the reversal of insulin resistance, sarcopenia and metabolic syndrome in an afflicted individual in need thereof (such as a patient with polycystic ovary syndrome or NASH), comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present invention provides the use (or method, said methods do not form part of the present invention) of the therapeutic agent A and the therapeutic agent B in the preparation of a medicament for the treatment of nonalcoholic fatty liver disease (NAFLD) or nonalcoholic steatohepatitis (NASH) in an afflicted individual in need thereof, where the therapeutic agent A is a non-steroidal anti-inflammatory drug according to the claims, and the therapeutic agent B is a fatty acid oxidation inhibitor according to the claims. In some embodiments, the present invention provides the use (or method, said methods do not form part of the present invention) for the treatment of nonalcoholic fatty liver disease (NAFLD) or nonalcoholic steatohepatitis (NASH), comprising administering the therapeutic agent A and the therapeutic agent B to an afflicted individual, where the therapeutic agent A is a non-steroidal anti-inflammatory drug according to the claims, and the therapeutic agent B is a fatty acid oxidation inhibitor according to the claims. The therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for curbing weight gain (or reverse hyperlipidemia) in an afflicted individual in need thereof (such as a patient with nonalcoholic fatty liver disease (NASH)), where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) for curbing weight gain (or reverse hyperlipidemia) in an afflicted individual in need thereof (such as a patient with nonalcoholic fatty liver disease (NASH)), comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for the reversal of liver enlargement (or liver damage) in an afflicted individual in need thereof (such as a patient with nonalcoholic fatty liver disease (NASH)), where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) for the reversal of liver enlargement (or liver damage) in an afflicted individual in need thereof (such as a patient with nonalcoholic fatty liver disease (NASH)), comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for increasing glucose tolerance (or reversing insulin resistance, restoring skeletal muscle and liver insulin sensitivity, and relieving muscle inflammation) in an afflicted individual in need thereof (such as a patient with nonalcoholic fatty liver disease (NASH)), where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) for increasing glucose tolerance (or reversing insulin resistance, restoring skeletal muscle and liver insulin sensitivity, and relieving muscle inflammation) in an afflicted individual in need thereof (such as a patient with nonalcoholic fatty liver disease (NASH)), comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for promoting angiogenesis (or promoting neuromuscular tissue sensitivity) in an afflicted individual in need thereof (such as a patient with nonalcoholic fatty liver disease (NASH)), where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) for promoting angiogenesis (or promoting neuromuscular tissue sensitivity) in an afflicted individual in need thereof (such as a patient with nonalcoholic fatty liver disease (NASH)), comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

In some embodiments, the present disclosure provides the use (or method, said methods do not form part of the present invention) of the therapeutic agent A and the therapeutic agent B in the preparation of a medicament for the modulation of p38 and AMPK signaling pathways in an afflicted individual in need (such as a patient with nonalcoholic fatty liver disease (NASH)), where the therapeutic agent A is a non-steroidal anti-inflammatory drug, and the therapeutic agent B is a fatty acid oxidation inhibitor. In some embodiments, the present disclosure provides the use (or method, said methods do not form part of the present invention) for the modulation of p38 and AMPK signaling pathways in an afflicted individual in need (such as a patient with nonalcoholic fatty liver disease (NASH)), comprising administering the therapeutic agent A and the therapeutic agent B to an afflicted individual, where the therapeutic agent A is a non-steroidal anti-inflammatory drug, and the therapeutic agent B is a fatty acid oxidation inhibitor. In some embodiments, the therapeutic agent A is selected from salicylates (e.g., aspirin) and the therapeutic agent B is selected as trimetazidine. In some embodiments, the therapeutic agent A (a salicylate, such as aspirin) and the therapeutic agent B (trimetazidine) are in a weight ratio of from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1.

The definition of the therapeutic agent A and the therapeutic agent B, as well as the administration frequency, mode and dosage, etc., may be the same as previously described.

In some embodiments, the therapeutic agent A is selected from salicylates and the therapeutic agent B is selected as trimetazidine. In some embodiments, the therapeutic agent A is selected as aspirin and the therapeutic agent B is selected as trimetazidine. In some embodiments, the weight ratio of the therapeutic agent A (a salicylate, such as aspirin) to the therapeutic agent B (trimetazidine) is from 1:1 to 10:1, including 2:1, 3:1, 4:1, 5:1, 6:1, 7:1, 8:1, 9:1, or 10:1. In some embodiments, the weight ratio of the therapeutic agent A (a salicylate, such as aspirin) to the therapeutic agent B (trimetazidine) is 6:1. In some embodiments, the therapeutic agent A and the therapeutic agent B are administered concurrently (e.g., orally). In some embodiments, the therapeutic agent A and the therapeutic agent B are contained in a single dosage form. In some embodiments, the single pharmaceutical dosage form is an oral dosage form. In some embodiments, the therapeutic agent A and the therapeutic agent B are administered separately (e.g., orally).

A course of treatment may include 1, 2, 3, 4, 5, 6, 7, 8, 9, 10, 11, 12 months or longer. In some embodiments, the therapeutic agent A may be administered once a day, twice a day, three times a day or the like, or may be administered once every two days, once every three days, once a week, or at other frequencies. In some embodiments, the therapeutic agent B may be administered once a day, twice a day, three times a day or the like, or may be administered once every two days, once every three days, once a week, or at other frequencies. When the therapeutic agent A and the therapeutic agent B are administered separately, the frequency of administration of the two may or may not be the same.

According to the present disclosure, the subjects in need thereof can be divided into many types, for example, subjects who have been diagnosed with or are at risk for various metabolic syndromes, or subjects whose indicators of various metabolic syndromes are out of the normal range, or subjects who are intolerant, unavailable, or ineffective after use of certain antidiabetic drugs, etc. Thus, in an embodiment, the subject may be an individual diagnosed with one or more conditions selected from the group consisting of overweight, obesity, visceral obesity, and abdominal obesity. In another embodiment, the subject may be an individual diagnosed with one or more of the following conditions: (a) fasting blood glucose or serum glucose concentration is greater than 110 mg/dL, especially greater than 125 mg/dL; (b) postprandial plasma glucose concentration is equal to or greater than 140 mg/dL; and (c) HbA1c value is equal to or greater than 6.5%, in particular equal to or greater than 8.0%. In another embodiment, the subject may be an individual with one or more of the following conditions: (a) obesity, visceral obesity and/or abdominal obesity; (b) triglyceride blood concentration ≥ 150 mg/dL; (c) HDL-cholesterol blood level < 40 mg/dL in female subjects; HDL-cholesterol blood level < 50 mg/dL in male subjects; (d) systolic blood pressure ≥ 130 mmHg, diastolic blood pressure ≥ 85 mmHg; (e) fasting blood glucose level ≥ 110 mg/dL; and (f) LDL-cholesterol blood levels ≥ 130 mg/dL. In another embodiment, the subject may be an individual who is contraindicated in metformin monotherapy and/or intolerant to therapeutic doses of metformin. In another embodiment, the subject may be an individual with insufficient glycemic control after treatment with one or more antidiabetic drugs selected from the group consisting of: (a) a biguanide; (b) a sulfonylurea; (c) a meglitinide; (d) a thiazolidinedione; (e) an α-glucosidase inhibitor; (f) insulin and an insulin analog; (g) a dipeptidyl peptidase IV inhibitor; (h) an SGLT2 inhibitor; (i) a PPARα modulator; (j) a glucose-dependent insulinotropic polypeptide agonist; (k) a β-3 agonists; (l) GLP1 and a GLP1 analog; (m) a PPARγ modulator; and (n) an HMG-CoA reductase inhibitor.

In some embodiments, the subject is a human, such as a person who is over the age of 20, 30, 40, 50, 60, 70, or 80.

The present disclosure will be described in detail below with reference to examples.

### Examples

In the following examples, all animal (such as mice) procedures were performed based on the animal care guidelines approved by the Institutional Animal Care and Use Committee. For histology, tissue samples have been fixed in 10% neutral formalin or Bouin solution and then embedded in paraffin.

In addition, the control group and the experimental group of the present invention all adopt intraperitoneal injections in the process. The injection volume starts at 4 µl/g of body weight and can be adjusted according to the dose, in which the therapeutic agent A in the experimental group (aspirin (trade name Aspirin) is used in the following examples) is diluted in PBS to a dose between 0.3 mg/kg and 120 mg/kg of body weight, the therapeutic agent B (trimetazidine (trade name Trimetazidine) is used in the following examples) is diluted in PBS to a dose between 0.05 mg/kg and 500 mg/kg of body weight. In the control group, the same amount of the therapeutic agent A or the same amount of the therapeutic agent B alone is usually used, or only the same amount of PBS solvent is used as a blank control.

For the results in each graph, data are expressed as mean ± standard error of mean (SEM) and Student's t-test (two-tailed, two-sample unequal variance) has been used to calculate p values. Statistical significance is shown as p>0.05 (no significance, ns), p<0.05 (one asterisk, i.e. *), or p<0.01 (two asterisks, i.e. **), or p<0.001 (three asterisks, i.e. ***). Tests are performed using Microsoft Excel, where the test type is always set to Two-Sample Equal Variance.

### Example 1

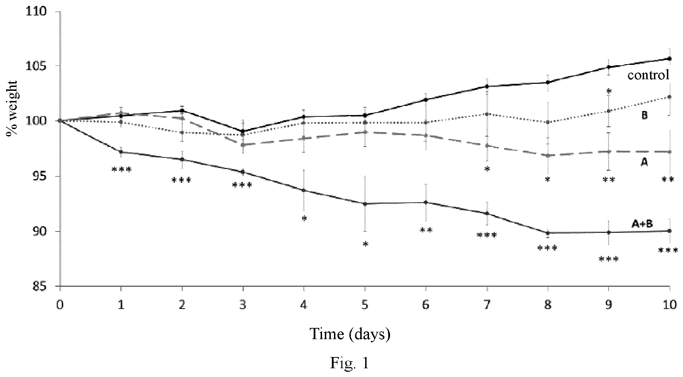

Twenty 6-week-old diet-induced obesity (Jackson Lab's Diet-Induced Obese, DIO) male mice with similar physical status were selected and then divided into four groups, and the number of mice in each group was 5. For the experimental group, each mouse was effectively administered 30 mg/kg of therapeutic agent A and 5 mg/kg of therapeutic agent B per day; for control group 1, each mouse was effectively administered 30 mg/kg of therapeutic agent A per day; for control group 2, each mouse was effectively administered 5 mg/kg of therapeutic agent B per day; for control group 3, only the same amount of PBS solvent was administered to each mouse every day as a blank control. The experiment was conducted for 10 days in total. The body weight of each mouse was recorded daily. A graph of body weight over time was plotted based on the body weight of each mouse, as shown in Fig. 1.

Fig. 1 shows the results of weight changes in induced obese DIO mice by the pharmaceutical therapeutic agents A+B in the experimental group treated with a pharmaceutical composition according to an embodiment of the present invention, as compared to the control groups. It can be seen that the pharmaceutical composition of the present invention can effectively reduce the body weight of DIO mice by more than about 10% within 10 days; while the control groups 1 to 3 had almost no significant effect on the body weight of the mice. Therefore, it can be considered that the therapeutic agent A and the therapeutic agent B provided by the present invention have a synergistic effect on the therapeutic effect of weight loss when used in combination.

### Example 2

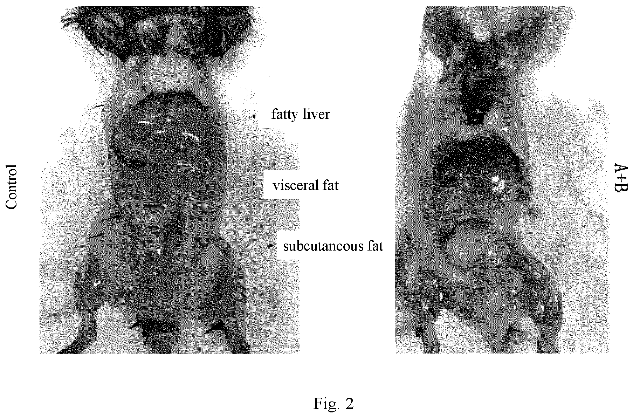

The mice in the experimental group and control group 3 that have completed Example 1 were selected and then dissected to observe the accumulation of fat around the mice in the experimental group and the control group 3, and a comparison was made. The captured images of fat deposits at various locations after dissection are shown in Fig. 2.

Fig. 2 shows the results of fat reduction in DIO mice induced by treating with the pharmaceutical therapeutic agents A+B in an experimental group using a pharmaceutical composition according to an embodiment of the present invention, as compared with the control group(s). It can be seen that there are many obvious fat accumulations in the mice in the control group 3, such as fatty liver, visceral fat and subcutaneous fat, while the fat accumulation in the mice in the experimental group is significantly reduced. Therefore, it can be considered that the pharmaceutical composition provided by the present invention has the effect of significantly reducing the content of body fat.

### Example 3

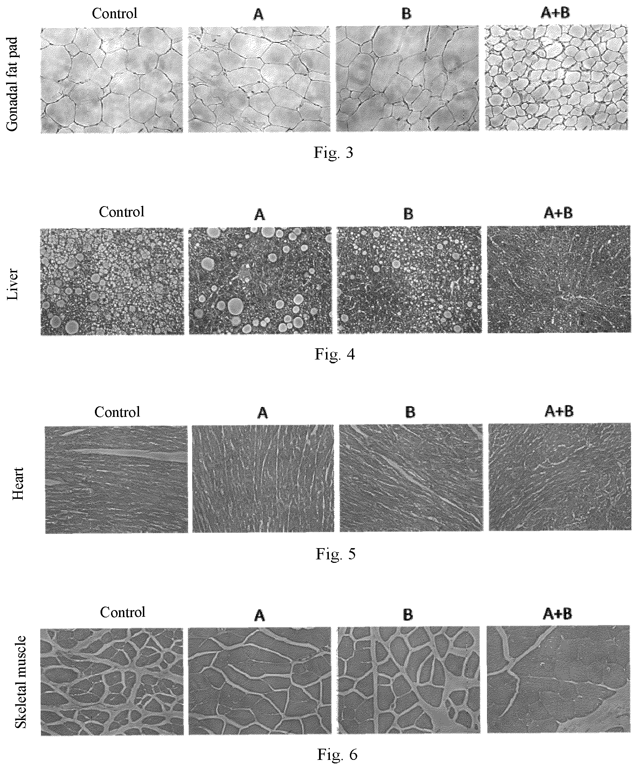

Similarly, the mice in the experimental group and control groups 1, 2, and 3 that had completed Example 1 were selected, and the dissected mice were observed under a microscope to compare the shape and size of fat granules in the gonadal fat pad. An illustration of the morphology of fat granules under the microscope is shown in Fig. 3.

Fig. 3 shows the results of the morphology of adipocytes in the gonadal fat pad in an experimental group treated with the pharmaceutical composition according to an embodiment of the present invention, as compared with the control groups. It can be seen that, compared with the fat granules in the control groups 1, 2, and 3, the size of the fat granules in the experimental group was significantly reduced, that is to say, the therapeutic agent A+B had the therapeutic effect of inducing fat reduction.

### Example 4

Similarly, the mice in the experimental group and control groups 1, 2, and 3 that had completed Example 1 were selected, and the dissected mice were observed under a microscope to compare the morphology and fat granule size of liver cells. An illustration of the morphology of liver cells under the microscope is shown in Fig. 4.

Fig. 4 shows the results of fatty liver and liver cell morphology in an experimental group treated with the pharmaceutical composition according to an embodiment of the present invention, as compared with the control groups. It can be seen that compared with the liver cells in the control groups 1, 2, and 3, the number of fat granules in the liver cells in the experimental group was reduced, the size thereof was significantly reduced, and the inflammatory cells were reduced as well. That is to say, the therapeutic agent A+B had the therapeutic effect of inducing the reduction of fat and inflammation in fatty liver.

### Example 5

Similarly, the mice in the experimental group and control groups 1, 2, and 3 that had completed Example 1 were selected, and the dissected mice were observed under a microscope to compare the cardiac muscle cell morphology. An illustration of the morphology of cardiac muscle cell under the microscope is shown in Fig. 5.

Fig. 5 shows the results of cardiac muscle cell morphology in an experimental group treated with the pharmaceutical composition according to an embodiment of the present invention, as compared with the control groups. It can be seen that the color and shape of the muscle images in the experimental group and the control group 1, 2, and 3 were basically the same. That is to say, the therapeutic agents A+B did not cause cardiotoxicity.

### Example 6

Similarly, the mice in the experimental group and control groups 1, 2, and 3 that had completed Example 1 were selected, and the dissected mice were observed under a microscope to compare the skeletal muscle cell morphology. An illustration of the morphology of skeletal muscle cells under the microscope is shown in Fig. 6.

Fig. 6 shows the results of skeletal muscle cell morphology in an experimental group treated with the pharmaceutical composition according to an embodiment of the present invention, as compared with the control groups. It can be seen that the color and shape of the muscle images in the experimental group and the control group 1, 2, and 3 were basically the same. That is to say, the therapeutic agents A+B did not cause muscle loss and muscle toxicity.

### Example 7

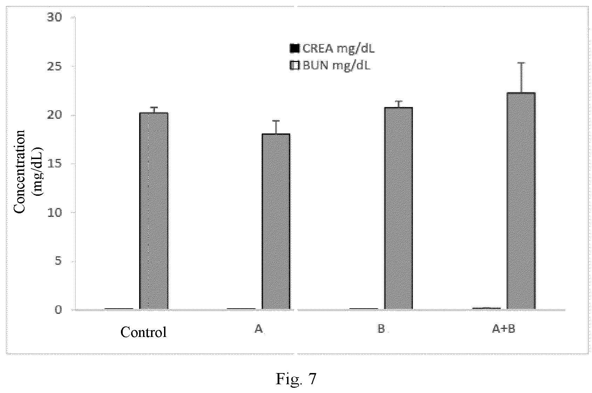

Similarly, the mice in the experimental group and control groups 1, 2, and 3 that had completed Example 1 were selected, and the sera of the dissected mice were used for blood biochemical tests to compare the renal toxicity indicators serum creatinine (CREA) and blood urea nitrogen (BUN). The comparison of the two indicators is shown in Fig. 7.

Fig. 7 shows the results of renal toxicity indexes serum creatinine (CREA) and blood urea nitrogen (BUN) in an experimental group treated with the pharmaceutical composition according to an embodiment of the present invention, as compared with the control groups. It can be seen that the serum creatinine (CREA) and blood urea nitrogen (BUN) levels in the experimental group and the control group 1, 2, and 3 were basically the same, and there was no significant difference therebetween. That is to say, the therapeutic agents A+B did not cause renal toxicity.

### Example 8

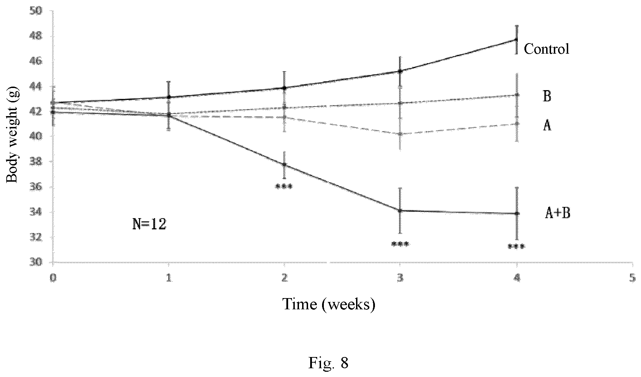

Diabetic DIO mice were tested for body weight change according to the same method as in Example 1, except that 12 mice were included in each group, and the duration of treatment was increased to 4 weeks. The body weight of each mouse was recorded daily, and the body weight of each mouse was plotted over time, as shown in Fig. 8.

Fig. 8 shows the results of weight changes in diabetic DIO mice after 4 weeks of pharmaceutical therapeutic agents A+B treatment in an experimental group treated with the pharmaceutical composition according to an embodiment of the present invention, as compared with the control groups. It can be seen that the pharmaceutical composition of the present invention can effectively and stably reduce the body weight of the diabetic DIO mice by more than about 20% within 3 weeks, while the control groups 1 to 3 have almost no obvious effect on the body weight of the mice. Therefore, it can be considered that the therapeutic agent A and the therapeutic agent B provided by the present invention, when used in combination, have a synergistic effect on the therapeutic result of weight loss in diabetic patients.

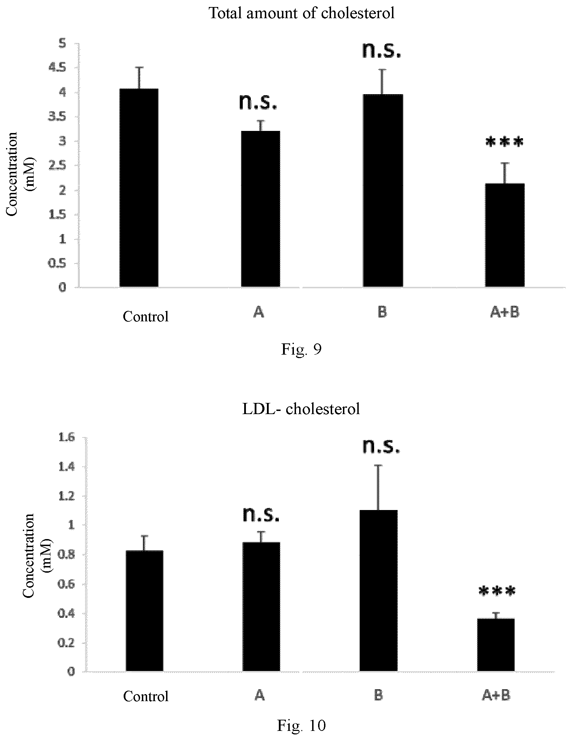

Fig. 9 shows the comparison of the total amount of serum cholesterol, an indicator associated with hyperlipidemia, in each group of DIO mice.

Fig. 10 shows the comparison of serum LDL-cholesterol, a marker associated with hyperlipidemia, in each group of DIO mice.

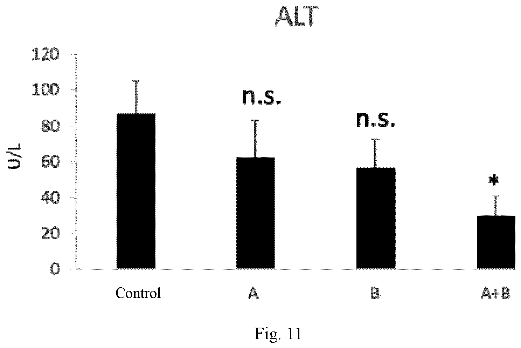

Fig. 11 shows the comparison of serum alanine aminotransferase (ALT), an indicator associated with fatty liver, in each group of DIO mice.

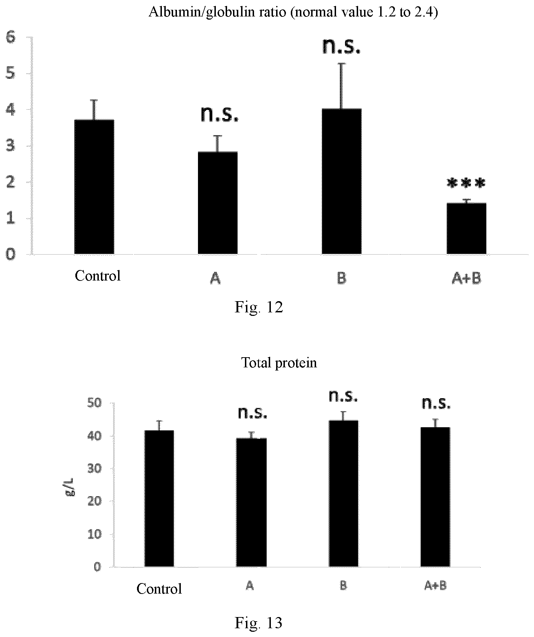

Fig. 12 shows the comparison of serum albumin/globulin ratio, an indicator associated with nitrogen metabolism function, in each group of DIO mice.

Fig. 13 shows the comparison of serum total protein, an indicator associated with nitrogen metabolism function, in each group of DIO mice.

### Examples 9 to 13

Subsequent tests were performed on each group of mice that had completed the treatment of Example 8. Examples 9 and 10 detected two indicators related to obesity and hyperlipidemia: total cholesterol and LDL-cholesterol; Example 11 detected alanine aminotransferase (ALT), an indicator related to fatty liver disease; and Examples 12 and 13 detected two indicators related to nitrogen metabolism: albumin/globulin ratio and total protein. The comparison of each indicator for each group is shown in Figs 9 to 13.

Figs 9 to 13 show the comparison of various indicators related to metabolic syndrome such as obesity, hyperlipidemia, fatty liver diseases, and nitrogen metabolism function in each group of mice, respectively. It can be seen that after the administration of the therapeutic agent A+B for 4 weeks, compared with the blank control group 3, the indicators in the experimental group mice all showed a significant decrease, while neither therapeutic agent A in control group 1 nor therapeutic agent B in control group 2 used alone was able to achieve significant therapeutic or restorative effects. It can be seen that the pharmaceutical composition provided by the present invention has obvious medical application in the treatment or improvement of diseases related to obesity and hyperlipidemia, fatty liver diseases, and diseases related to nitrogen metabolism function.

### Example 14

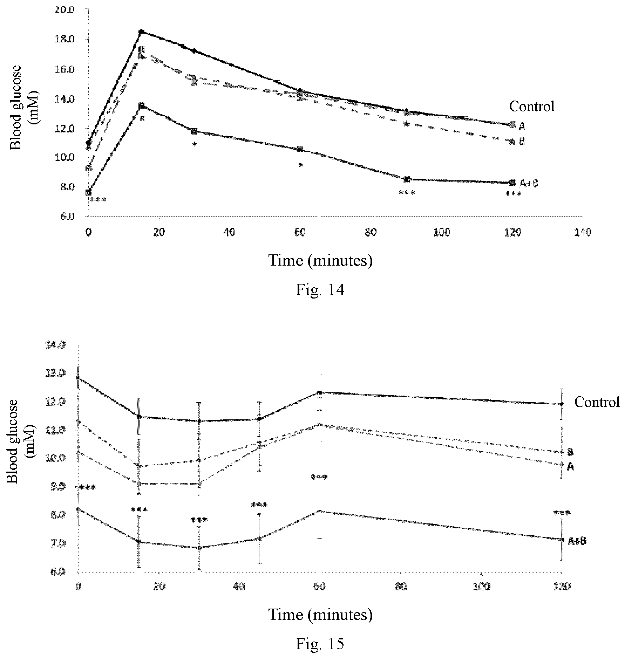

Similarly, glucose tolerance tests were also performed on groups of mice that had completed the treatment of Example 8. In the experimental group and the control groups 1 to 3, the experiment was performed by intraperitoneal glucose injection (2 mg/g of body weight) to mice fasted overnight. After every 15 to 30 minutes, the glucose content (mM) in the mice was tested at each time point with a LifeScan OneTouch^{®} blood glucose meter, and a line graph was drawn according to the glucose content and time. The results are shown in Fig. 14.

Fig. 14 shows the results of glucose tolerance in diabetic DIO mice. It can be seen that, compared with the control groups 1 to 3, the glucose content of the mice in the experimental group did not significantly increase to a very high level of glucose content, and it quickly decreased to a lower level of glucose content. Thus, it can be seen that the mice showed excellent performance in glucose tolerance after 4 weeks of treatment with the pharmaceutical composition of the present invention.

### Example 15

Similarly, insulin sensitivity tests were also performed on groups of mice that had completed the treatment of Example 8. In the experimental group and control groups 1 to 3, 0.75 U insulin/kg of body weight of insulin (Humulin) was administered intraperitoneally to 5-hour fasted rats by using a 27G syringe needle. Every 15-30 minutes thereafter, mice were tested for glucose levels (mM) at each time point with a LifeScan OneTouch^{®} glucometer, and a line graph is plotted based on glucose content and time. The results are shown in Fig. 15.

Fig. 15 shows the results of insulin sensitivity in diabetic DIO mice. It can be seen that, compared with the control groups 1 to 3, the glucose content of the mice in the experimental group was always kept at a lower level of glucose content and changed relatively more significantly. It can thus be seen that the mice after 4 weeks of treatment with the pharmaceutical composition of the present invention have exhibited excellent performance in terms of insulin sensitivity.

### Example 16

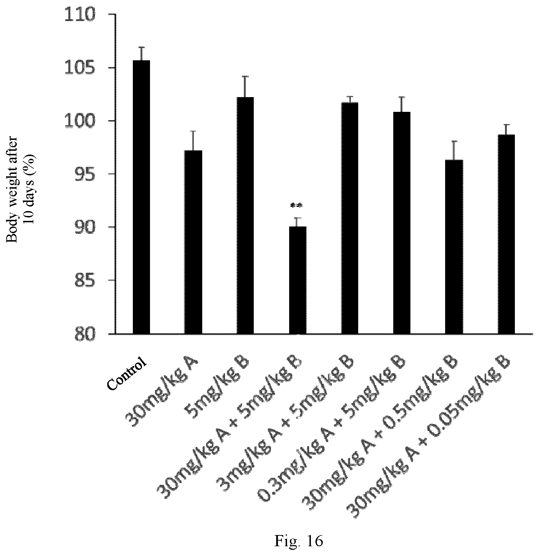

Different combinations of therapeutic agents were used to treat the DIO mice for 10 days as follows: (1) blank control; (2) 30 mg/kg A; (3) 5 mg/kg B; (4) 30 mg/kg A+5 mg/kg B; (5) 3 mg/kg A+5 mg/kg B; (6) 0.3 mg/kg A+5 mg/kg B; (7) 30 mg/kg A+0.5 mg/kg B; (8) 30 mg/kg A +0.05 mg/kg B. At the end of the treatment, the body weight (%) in the group of each combination of therapeutic agents was measured, respectively. The results are shown in Fig. 16.

Fig. 16 shows the comparison of body weights in DIO mice following administration of various therapeutic agent combinations. It can be seen that the expected therapeutic effect cannot be achieved in the case of administering the usual doses. In contrast, in the case of the therapeutic agent combination of 30 mg/kg A+5 mg/kg B in group (4), excellent performance in weight loss has been exhibited.

### Example 17

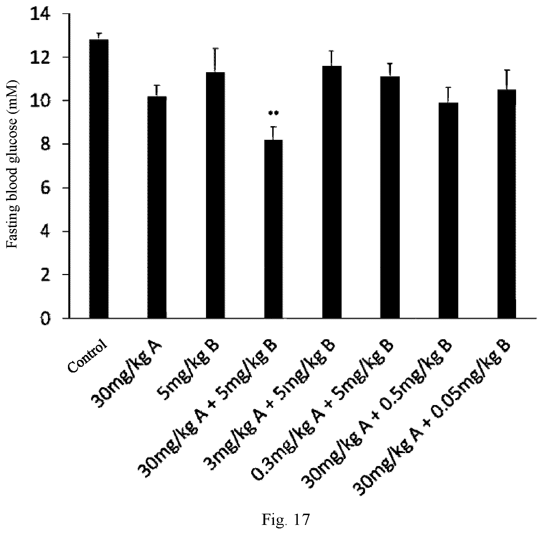

Different combinations of therapeutic agents were used to treat the DIO mice for 10 days as follows: (1) blank control; (2) 30 mg/kg A; (3) 5 mg/kg B; (4) 30 mg/kg A+5 mg/kg B; (5) 3 mg/kg A+5 mg/kg B; (6) 0.3 mg/kg A+5 mg/kg B; (7) 30 mg/kg A+0.5 mg/kg B; (8) 30 mg/kg A +0.05 mg/kg B. At the end of the treatment, fasting blood glucose levels (mM) were measured in each therapeutic agent combination group, and the results are shown in Fig. 17.

Fig. 17 shows the comparison of fasting blood glucose levels in DIO mice following administration of various therapeutic agent combinations. It can be seen that the expected therapeutic effect cannot be achieved in the case of administering the usual doses. In contrast, in the case of the therapeutic agent combination of 30 mg/kg A+5 mg/kg B in group (4), excellent performance in reducing fasting blood glucose levels has been exhibited.

### Example 18

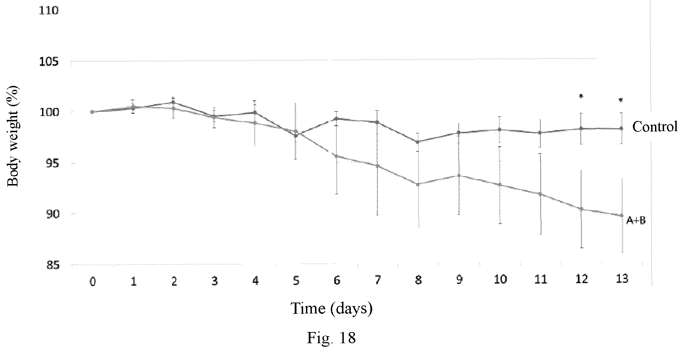

Common wild-type C57BL/6 mice with similar physical status in all aspects were selected in the test. For the experimental group, 30 mg/kg of therapeutic agent A and 5 mg/kg of therapeutic agent B were effectively administered to each mouse per day; for the blank control group, only the same amount of PBS solvent was administered to each mouse per day. The experiment was conducted 13 days in total. The body weight of each mouse was recorded daily, and the body weight of each mouse was plotted over time as shown in Fig. 18.

Fig. 18 shows the results of body weight change in common wild-type C57BL/6 mice induced by the pharmaceutical therapeutic agents A+B in an experimental group treated with the pharmaceutical composition according to an embodiment of the present invention, as compared with the control groups. It can be seen that the pharmaceutical composition of the present invention can effectively reduce the body weight of common wild-type C57BL/6 mice by more than 10% within 13 days, while the control group has almost no obvious effect on the body weight of the mice. Therefore, it can be considered that the therapeutic agent A and the therapeutic agent B provided by the present invention also have a synergistic effect on the therapeutic result of weight loss in common wild-type C57BL/6 mice when used in combination.

### Example 19

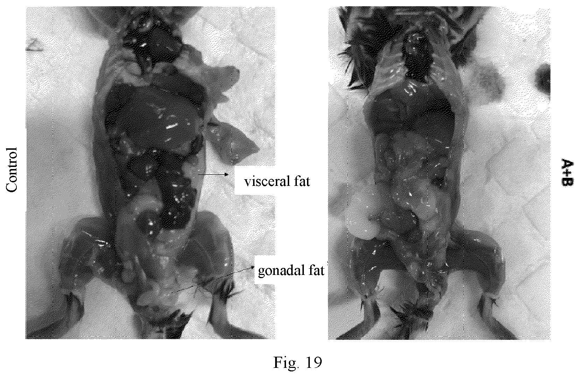

The mice in the experimental group and the control groups that have completed Example 18 were selected and dissected to observe the accumulation of fat in the mice in the experimental group and in the control groups. Comparisons were then made therebetween, and the captured images of the fat deposits at various locations after dissection are shown in Fig. 19.

Fig. 19 shows the results of fat reduction in common wild-type C57BL/6 mice induced by the pharmaceutical therapeutic agents A+B in an experimental group treated with the pharmaceutical composition according to an embodiment of the present invention, as compared with the control groups. It can be seen that there are many obvious fat accumulations in the mice in the control groups, such as visceral fat and gonadal fat, while the fat accumulation in the mice in the experimental group is significantly reduced. Therefore, it can be considered that the pharmaceutical composition provided by the present invention has the effect of significantly reducing the body fat content.

### Example 20

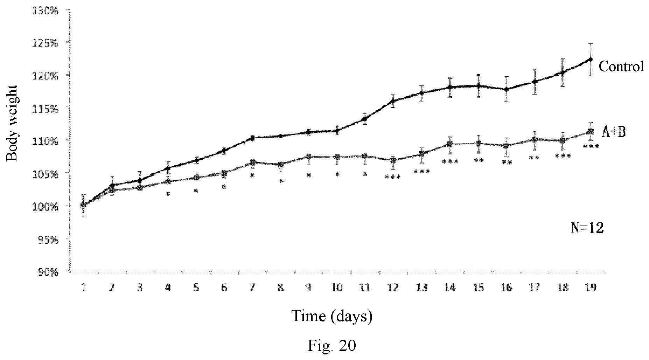

Twenty-four 8-week-old Sprague Dawley female rats with similar physical status were selected and divided into two groups. The number of rats in each group was twelve. Each rat was fed a daily high-fat, high-sugar diet (HFHSD, D11092103; Research Diet Inc) and daily subcutaneous injection of 60 mg/kg dehydroepiandrosterone (DHEA; Sigma Aldrich) to mimic polycystic ovary syndrome (PCOS) (Zhang et al., Reproduction 2016). For the experimental group, each rat was effectively administered 30 mg/kg of therapeutic agent A and 5 mg/kg of therapeutic agent B per day; for the control group, the same amount of PBS solvent was effectively administered to each rat every day as a blank control. The experiment was conducted for three weeks. The body weight of each rat was recorded daily, and the body weight of each rat was plotted over time, as shown in Fig. 20.

Fig. 20 shows the results of body weight change in polycystic ovary syndrome rats induced by the pharmaceutical therapeutic agents A+B in an experimental group treated with the pharmaceutical composition according to an embodiment of the present invention, as compared with the control group. It can be seen that the pharmaceutical composition of the present invention can effectively and stably avoid more than about 10% body weight gain of polycystic ovary syndrome rats within 3 weeks. Therefore, it can be considered that the therapeutic agent A and the therapeutic agent B provided by the present invention, when used in combination, have a synergistic effect on the prevention of obesity in patients with polycystic ovary syndrome.

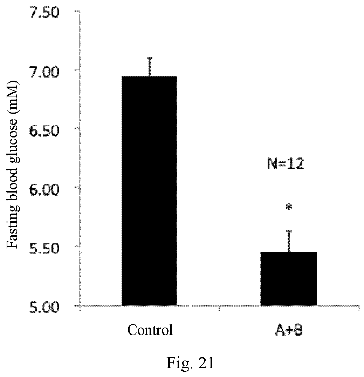

Fig. 21 shows the comparison of fasting blood glucose levels in each group of polycystic ovary syndrome rats.

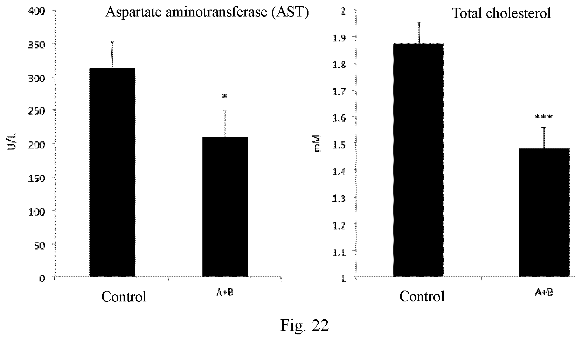

Fig. 22 shows the comparison of serum aspartate aminotransferase (AST) and total cholesterol, indicators associated with fatty liver, in each group of polycystic ovary syndrome rats.

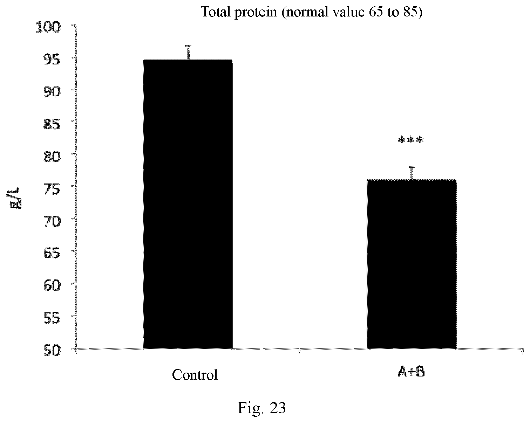

Fig. 23 shows the comparison of serum total protein, an indicator associated with liver nitrogen metabolism function, in each group of polycystic ovary syndrome rats.

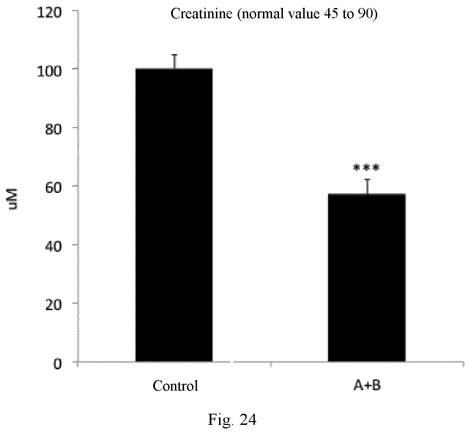

Fig. 24 shows the comparison of serum creatinine, an indicator associated with renal function, in each group of polycystic ovary syndrome rats.

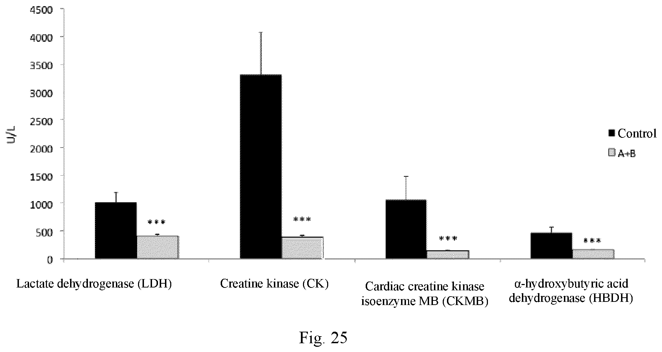

Fig. 25 shows the comparison of serum lactate dehydrogenase (LDH), creatine kinase (CK), cardiac creatine kinase isoenzyme MB (CKMB), α-hydroxybutyric acid dehydrogenase (HBDH), indicator associated with heart diseases, in each group of polycystic ovary syndrome rats.

### Examples 21 to 25

Subsequent tests were performed on each group of rats that had completed the treatment of Example 20. Example 21 detected fasting blood glucose, an indicator related to diabetes; Example 22 detected the indicators related to fatty liver disease, serum aspartate aminotransferase (AST) and total cholesterol; Example 23 detected the index serum total protein related to liver nitrogen metabolism function; Example 24 detected serum creatinine (creatinine), an indicator related to renal function; and Example 25 detected the indicators related to heart disease serum lactate dehydrogenase (LDH), creatine kinase (CK), cardiac creatine kinase isoenzyme MB (CKMB), α-hydroxybutyrate dehydrogenase ( BBDH). The comparison of each index of each group is shown in Figs. 21 to 25, respectively.

Figs. 21 to 25 respectively show the comparison of various indicators related to polycystic ovary syndrome such as diabetes, obesity, hyperlipidemia, fatty liver disease, and heart disease in each group of rats. It can be seen that after the administration of the therapeutic agent A+B for 3 weeks, compared with the blank control group, all the indicators in the rats in the experimental group have showed a significant decrease. It can be seen that the pharmaceutical composition provided by the present invention has obvious medical application in preventing or improving polycystic ovary syndrome and related diabetes, obesity, hyperlipidemia, fatty liver disease, and heart disease-related diseases.

### Example 26

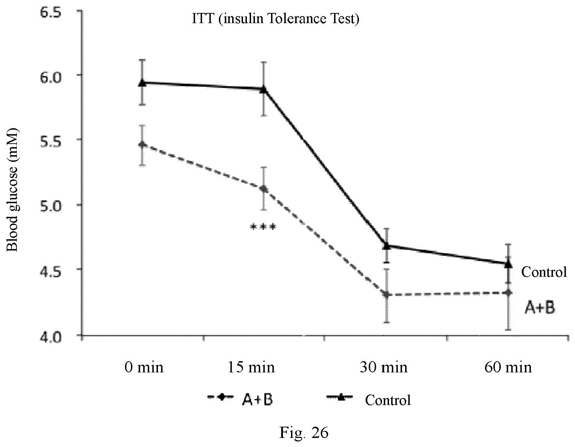

Similarly, insulin sensitivity tests were also performed on groups of rats that had completed the treatment of Example 20. In the experimental group and control the group, 0.75 U insulin/kg of body weight of insulin (Humulin) was administered intraperitoneally to 5-hour fasted rats by using a 27G syringe needle. Every 15-30 minutes thereafter, rats were tested for glucose levels (mM) at each time point with a LifeScan OneTouch^{®} glucometer, and a line graph is plotted based on glucose content and time. The results are shown in Fig. 26.

Fig. 26 shows the results of the insulin sensitivity in polycystic ovary syndrome rats. It can be seen that, compared with the control group, the glucose content of the rats in the experimental group was always kept at a lower level of glucose content and changed relatively more significantly. It can thus be seen that the rats after 3 weeks of treatment with the pharmaceutical composition of the present invention have exhibited excellent performance in terms of insulin sensitivity.

### Example 27

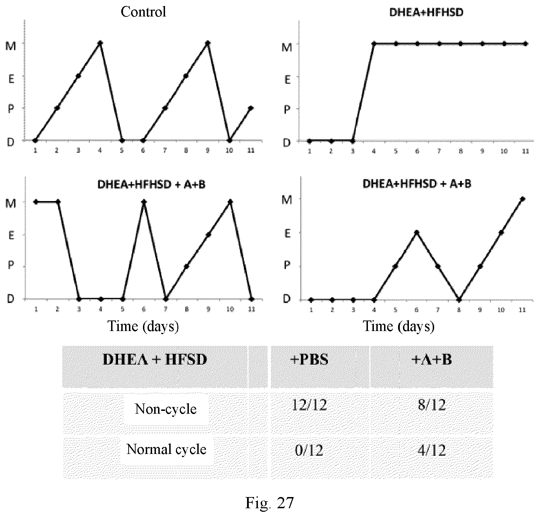

Similarly, vaginal cytology analysis was also performed on each group of rats of Example 20. Evaluation was performed with all stages of the estrus cycle for 11 consecutive days, including D interestrus, P preestrus, E estrus, and M late estrus. A line graph was drawn according to the estrus cycle stage and time, and the results are shown in Fig. 27.

Fig. 27 shows the results of the estrous cycle in polycystic ovary syndrome rats. It can be seen that, compared with the normal control group (Control), the estrous cycle of the polycystic ovary syndrome rats (DHEA+HFHSD) is abnormal, and 12/12 rats have non-cycle issue. The estrous cycle of rats in the experimental group (DHEA+HFHSD+A+B) was relatively normal, and the estrous cycle of 4/12 rats returned to normal completely. Therefore, it can be seen that after 3 weeks of treatment with the pharmaceutical composition of the present invention, some of the rats with polycystic ovary syndrome can completely recover the normal estrous cycle.

### Example 28

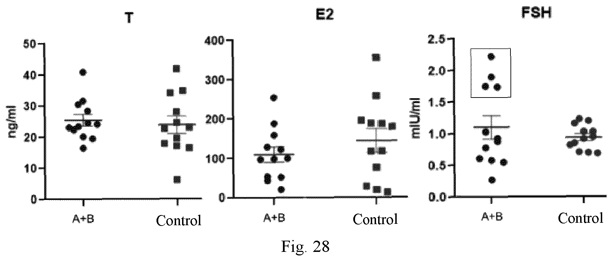

Similarly, ELISA analysis of serum hormones, including androgens (T), estrogens (E2), and follicle-stimulating hormone (FSH), were also performed on each group of the rats of Example 20. The results are shown in Fig. 28.

Fig. 28 shows the results of the serum hormone ELISA in polycystic ovary syndrome rats. It can be seen that compared with the control group (Control) of rats with polycystic ovary syndrome, the experimental group (A+B) had no significant changes in androgens (T) and estrogens (E2); the follicle-stimulating hormone (FSH) was significantly increased in 4/12 rats. Therefore, it can be seen that after 3 weeks of treatment with the pharmaceutical composition of the present invention, some of the rats with polycystic ovary syndrome can recover the normal estrus cycle. This is due to the increase in the follicle-stimulating hormone (FSH), rather than the regulation of androgens (T) and estrogens (E2).

### Example 29

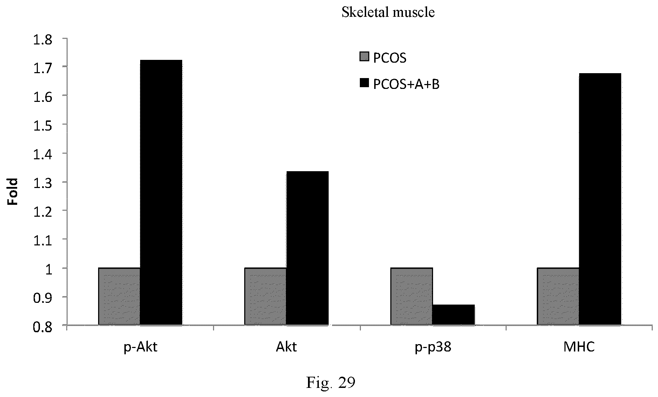

Similarly, Western blot analysis of skeletal muscle protein was also performed on each group of rats of Example 20, including phospho-Akt (S473), Akt, phospho-p38, muscle protein heavy chain (Myosin Heavy Chain, MHC), and GAPDH. The results are shown in Fig. 29.

Fig. 29 shows the results of Western blot analysis of skeletal muscle proteins in polycystic ovary syndrome rats. It can be seen that compared with the control group (PCOS) of the rats with polycystic ovary syndrome, the phospho-Akt (S473), Akt, Myosin Heavy Chain (MHC) were significantly increased, while phospho-p38 was significantly decreased. Therefore, it can be seen that after 3 weeks of treatment with the pharmaceutical composition of the present invention, the rats with polycystic ovary syndrome can restore normal skeletal muscle insulin sensitivity and protein synthesis and metabolism, and relieve muscle inflammation, insulin resistance, sarcopenia syndrome and metabolic syndrome.

### Example 30

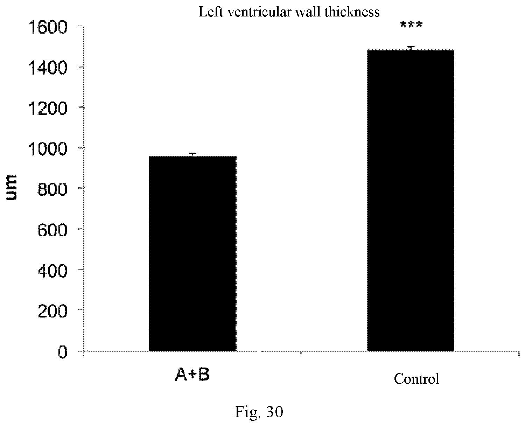

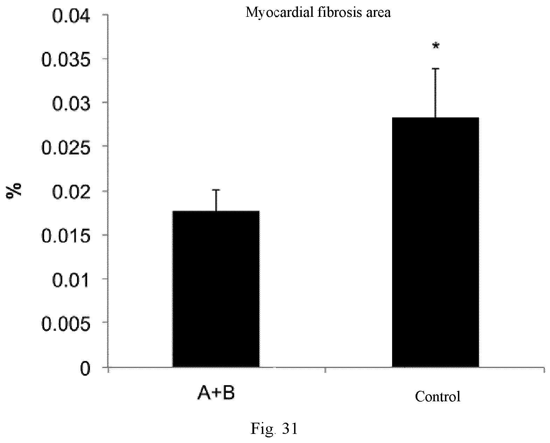

Similarly, cardiac section analysis, including ventricular wall thickness and Masson trichrome staining for fibrosis, was also performed on each group of rats of Example 20. The results are shown in Figs. 30 and 31.

Fig. 30 shows the results of left ventricular wall thickness analysis in polycystic ovary syndrome rats. It can be seen that, compared with the control group (Control) of the polycystic ovary syndrome rats, the left ventricular wall thickness of the experimental group (A+B) was significantly reduced (P<0.001). Therefore, it can be seen that the polycystic ovary syndrome rats treated with the pharmaceutical composition of the present invention for 3 weeks can significantly improve cardiovascular diseases such as ventricular hypertrophy and heart failure.

Fig. 31 shows the results of myocardial fibrosis analysis in polycystic ovary syndrome rats. It can be seen that the myocardial fibrosis area of rats in the experimental group (A+B) was significantly reduced compared with the control group (Control) of rats with polycystic ovary syndrome (P<0.05). Therefore, it can be seen that the polycystic ovary syndrome rats treated with the pharmaceutical composition of the present invention for 3 weeks can significantly improve cardiovascular diseases such as myocardial fibrosis, ventricular hypertrophy and heart failure caused by myocardial infarction.

### Example 32

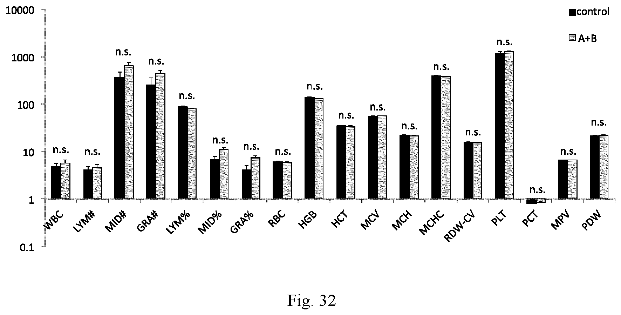

Similarly, blood routine analysis was also performed on each group of rats of Example 20, and the results are shown in Fig. 32.

Fig. 32 shows the results of routine blood analysis in polycystic ovary syndrome rats. It can be seen that compared with the control group (Control) of the rats with polycystic ovary syndrome, the blood routine indexes of the rats in the experimental group (A+B) have no significant changes (P>0.05). Therefore, it can be seen that after 3 weeks of treatment with the pharmaceutical composition of the present invention, the rats with polycystic ovary syndrome did not have significant toxic reactions.

### Example 33

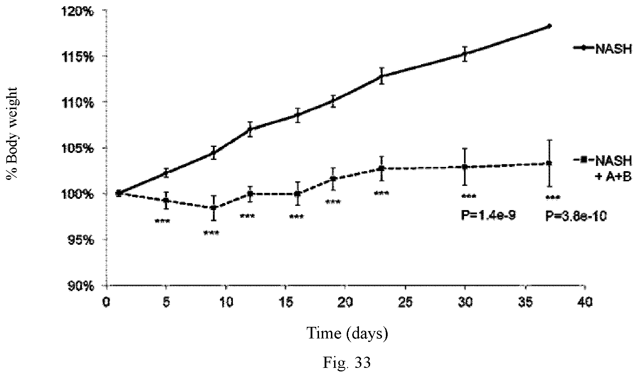

Twenty-six 6-week-old ob/ob obese (Jackson Lab B6.Cg-Lepob/J, Stock No: 000632) male mice from Jackson Laboratory with similar physical status were selected. Each mouse was fed a high-fat and high-sugar diet (HFSD, D11092103; Research Diet Inc) daily for 45 days to mimic fatty liver (NASH) (Kristiansen et al., 2016; do1:10.4254/wjh.v8.116.673 ). After 45 days, the mice were fed a high-fat and high-sugar diet continuously and were divided into two groups. The number of mice in each group was 13. For the experimental group, 30 mg/kg of the therapeutic agent A and 5 mg/kg of the therapeutic agent B were effectively administered to each mouse per day; for the control group, only the same amount of PBS solvent was administered to each mouse every day as a blank control for a total of 37 days. The body weight of each mouse was recorded daily, and the body weight of each mouse was plotted over time as shown in Fig. 33.

Fig. 33 shows the results of pharmaceutical therapeutic agents A+B on body weight change in NASH mice. It can be seen that, compared with the NASH control group, the pharmaceutical composition of the present invention can effectively reduce the body weight of NASH mice by more than about 3% within 5 days (P<0.001), and it was able to effectively reduce the body weight of NASH mice by about 20% in 37 days (P=3.8e-10), while 1-3 had almost no significant effect on the body weight of the mice. Therefore, it can be considered that the pharmaceutical therapeutic agent A+B provided by the present invention can effectively suppress weight gain.

### Example 34

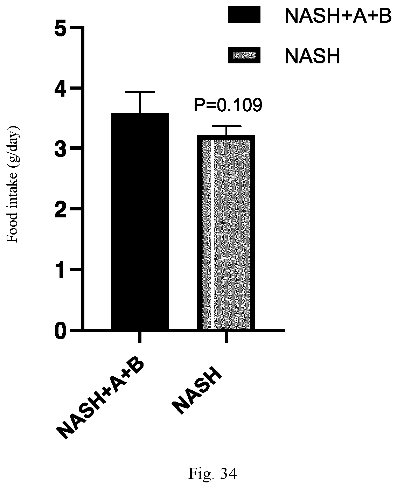

Similarly, groups of mice that had completed the treatment of Example 33 were also tested for three-day food intake, and the results are shown in Fig. 34.

Fig. 34 shows the results of the food intake test in NASH mice. It can be seen that, compared with the NASH control group, the food intake of the mice in the experimental group not only did not decrease, but was slightly increased (P=0.109). Therefore, it can be seen that there is no significant change in appetite of NASH mice after 40 days of treatment with the pharmaceutical composition of the present invention.

### Example 35

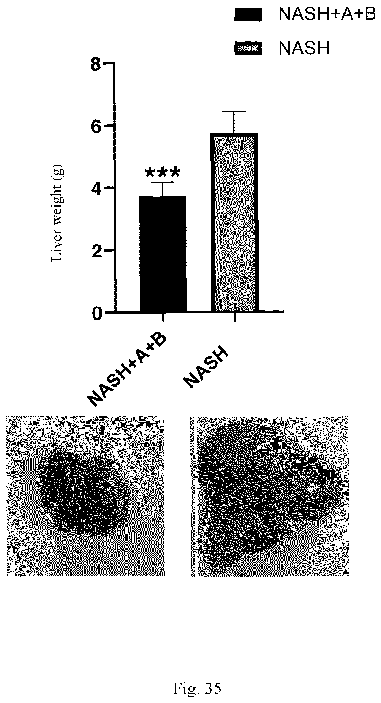

Similarly, each group of mice that had completed the treatment of Example 33 was also dissected, and liver weights were measured. The results are shown in Fig. 35.

Fig. 35 shows the results of the liver weight test in NASH mice. It can be seen that compared with the NASH control group, the liver weight of the mice in the experimental group was significantly reduced (P<0.001), and the liver volume was also significantly reduced. Therefore, it can be seen that NASH mice can reverse liver enlargement after 40 days of treatment with the pharmaceutical composition of the present invention.

### Example 36

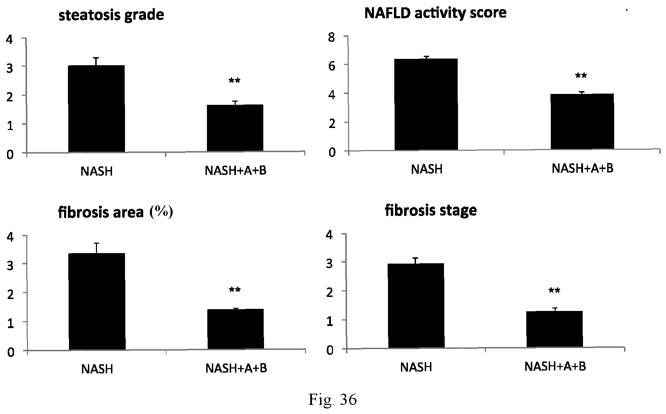

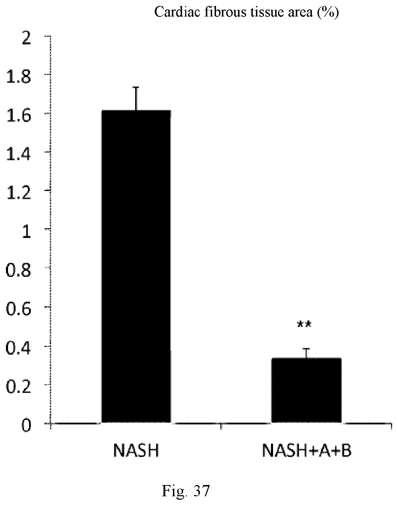

Similarly, each group of mice that had completed the treatment of Example 33 was also dissected, histologically fixed sectioned, stained with hematoxylin-eosin and Picro-Sirius red (Sigma-Aldrich), and subjected to microscopic observation and expert scoring. The liver analysis section results are shown in Fig. 36. The results of the cardiac analysis slices are shown in Fig. 37.

Fig. 36 shows the results of the liver section analysis in NASH mice. It can be seen that compared with the NASH control group, the fatty liver grade and fatty liver activity score of the mice in the experimental group were significantly reduced (P<0.01). That is to say, the therapeutic agent A+B has the therapeutic effect of inducing the reduction of fat and inflammation in fatty liver. At the same time, it can be seen that compared with the NASH control group, the liver fibrosis area (%) and fibrosis stage of the mice in the experimental group were also significantly reduced (P<0.01). That is to say, the therapeutic agent A+B has a therapeutic effect of inducing a reduction in fibrosis of fatty liver. After 40 days of treatment with the pharmaceutical composition of the present invention, the mice can reverse NASH fatty liver and liver fibrosis even though they continue to eat a large amount of high-fat and high-sugar feed.

Fig. 37 shows the results of the heart slice analysis in NASH mice. It can be seen that compared with the NASH control group, the cardiac fibrous tissue area of the mice in the experimental group was significantly reduced (P<0.01). That is to say, the therapeutic agent A+B has the therapeutic effect of inducing a reduction in cardiac fibrosis. Cardiovascular diseases such as cardiac fibrosis and heart failure can be reversed in mice treated with the pharmaceutical composition of the present invention for 40 days even though they continue to eat a large amount of high-fat and high-sugar feed.

### Example 38

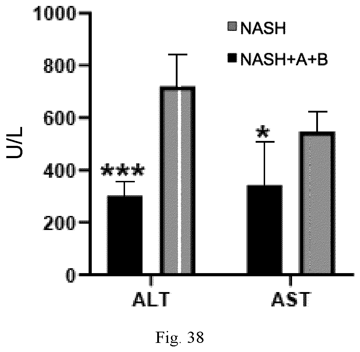

Similarly, blood biochemical tests were performed on each group of mice that had completed the treatment of Example 33. Example 38 detected indicators related to liver damage: alanine aminotransferase (ALT) and aspartate aminotransferase (AST); Example 39 examined the indicators associated with hyperlipidemia: triglyceride (TG), total cholesterol (CHOL), HDL-cholesterol (HDL-C) and LDL-cholesterol (LDL-C); Example 40 detected indicators related to various organ damage: albumin/globulin (Albumin/Globulin) ratio, urea (Urea), creatinine (Creatinine), lactate dehydrogenase (LDH), creatine kinase (CK), cardiac creatine kinase isoenzyme MB (CKMB) and α-hydroxybutyrate dehydrogenase (HBDH). The comparison of each indicator of each group is shown in Figs. 38 to 40, respectively.

Fig. 38 shows the comparison of two indicators associated with liver injury in each group of mice. Compared with the NASH control group, the alanine aminotransferase (ALT, P<0.001) and aspartate aminotransferase (AST, P=0.02) of the mice in the experimental group were significantly decreased. After 40 days of treatment with the pharmaceutical composition of the present invention, even though the mice continued to eat a large amount of high-fat and high-sugar diet, the liver damage could be reversed.

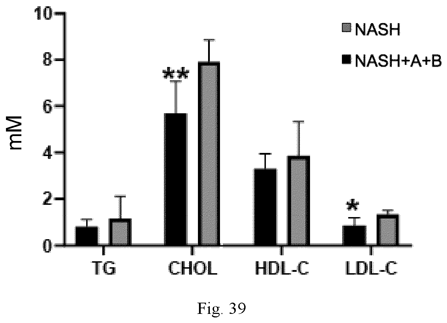

Fig. 39 shows the comparison of indicators associated with hyperlipidemia in each group of mice. Compared with the NASH control group, the triglyceride (TG), total cholesterol (CHOL, P<0.01), HDL-cholesterol (HDL-C) and LDL-cholesterol (LDL-C, P=0.016) of the mice in the experimental group were reduced. After 40 days of treatment with the pharmaceutical composition of the present invention, the mice could reverse hyperlipidemia even though they continued to eat a large amount of high-fat and high-sugar feed.

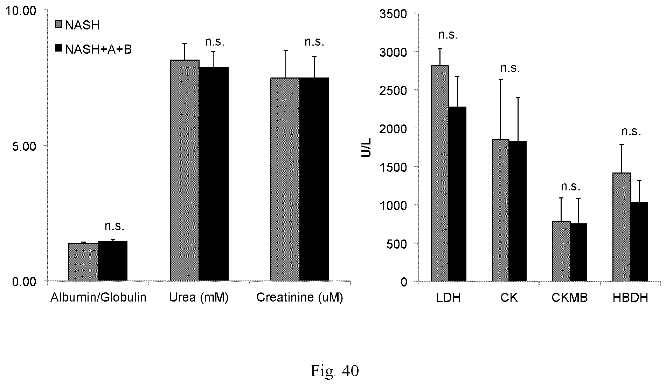

Fig. 40 shows the comparison of the indicators associated with organ damage in each group of mice. Compared with the NASH control group, the ratio of albumin/globulin, urea, creatinine, lactate dehydrogenase (LDH), creatine kinase (CK), urea (Urea), lactate dehydrogenase (LDH), creatine kinase (CK), cardiac creatine kinase isoenzyme MB (CKMB) and α-hydroxybutyrate dehydrogenase (HBDH) had no significant changes (P>0.05). After 40 days of treatment with the pharmaceutical composition of the present invention, various organs of the mice did not show any toxic reaction.

### Example 41

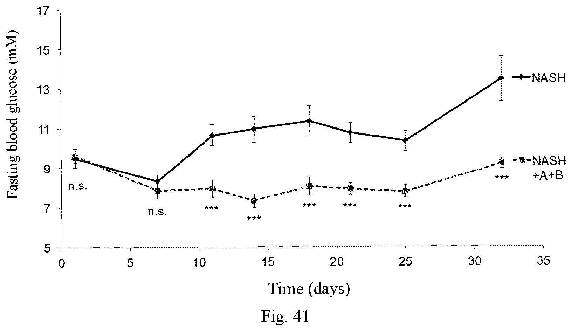

Similarly, each group of mice of Example 33 was tested for fasting blood glucose, the glucose content (mM) in mice fasted overnight was tested with a LifeScan OneTouch^{®} blood glucose meter every 3 to 7 days. A line graph was plotted based on the glucose content and time, and the results are shown in Fig. 41.

Fig. 41 shows the results of fasting blood glucose in each group of mice. It can be seen that compared with the NASH control group, the glucose content of the mice in the A+B experimental group not only did not increase significantly, but actually decreased significantly to a lower level of glucose content within 14 days (P<0.001). Therefore, it can be seen that the mice treated with the pharmaceutical composition of the present invention for 40 days have shown significant improvement in blood sugar control, and the occurrence of type 2 diabetes can be prevented.

### Example 42

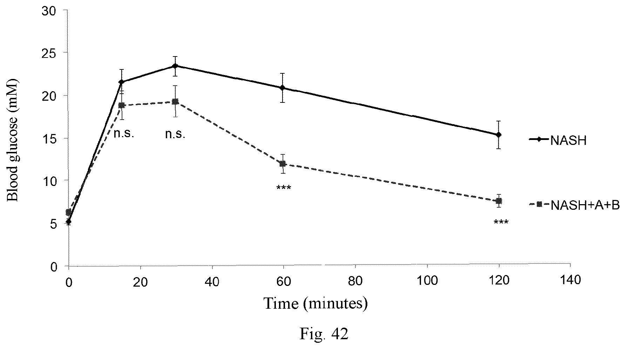

Similarly, each group of mice of Example 33 was also tested for glucose tolerance. In the experimental group and the control group, the test was performed by intraperitoneal injection of the same amount of glucose (2 mg/g of body weight) into the mice that fasted overnight. After every 15 to 30 minutes, the glucose content (mM) in the mice was tested at each time point with the LifeScan OneTouch^{®} blood glucose meter, and a line graph was drawn according to the glucose content and time. The results are shown in Fig. 42.

Fig. 42 shows the results of glucose tolerance in NASH mice. It can be seen that, compared with the NASH control group, the glucose content of the mice in the A+B experimental group increased to a level of glucose content that is not significantly high, and then quickly decreased to a lower level of glucose content (P<0.001). Thus, it can be seen that the NASH mice after 40 days of treatment with the pharmaceutical composition of the present invention have returned to normal in terms of glucose tolerance.

### Example 43

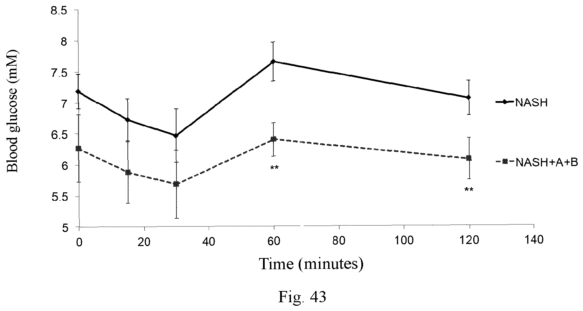

Similarly, each group of mice of Example 33 was tested for insulin sensitivity. In the experimental group and the control group, the same amount of insulin (Humulin, 0.75 U/kg of body weight) was intraperitoneally administered to rats fasted for 6 hours by using a 27G syringe needle. After every 15-30 minutes, the glucose content (mM) in the mice was tested at each time point with the LifeScan OneTouch^{®} blood glucose meter, and a line graph was drawn according to the glucose content and time. The results are shown in Fig. 43.

Fig. 43 shows the results of insulin sensitivity in NASH mice. It can be seen that, compared with the control group, the glucose content of the mice in the experimental group was always kept at a relatively low level and changed significantly after 60 minutes (P<0.01). Therefore, it can be seen that after 40 days of treatment with the pharmaceutical composition of the present invention, the NASH mice returned to normal in terms of insulin sensitivity, and the insulin resistance was reversed.

### Example 44

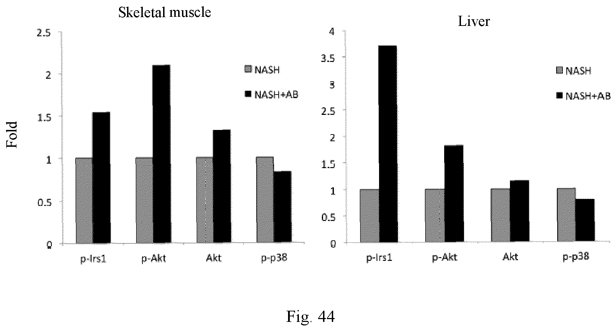

Similarly, the mice in each group of Example 33 were fasted for 6 hours and injected with insulin (Humulin, 0.75 U/kg body weight). After 15 minutes, skeletal muscle and liver samples were collected and protein Western blot analysis was performed to detect phospho-Irs1 (S307), phospho-Akt (S473), Akt, phospho-p38, and GAPDH. The results are shown in Fig.44.

Fig. 44 shows the results of Western blot analysis of skeletal muscle proteins in NASH mice. It can be seen that compared with the NASH mouse control group (Control), the skeletal muscle phospho-Irs1 (S307), phospho-Akt (S473), and Akt of the mice in the experimental group (A+B) were significantly increased, while p38 was decreased significantly. It can also be seen that compared with the NASH mouse control group (Control), the liver phospho-Akt (S473) and phospho-Irs1 (S307) of the mice in the experimental group (A+B) were significantly increased. Therefore, it can be seen that NASH mice treated with the pharmaceutical composition of the present invention for 40 days can restore normal skeletal muscle and liver insulin sensitivity, and relieve muscle inflammation, systemic insulin resistance and metabolic syndrome.

### Example 45

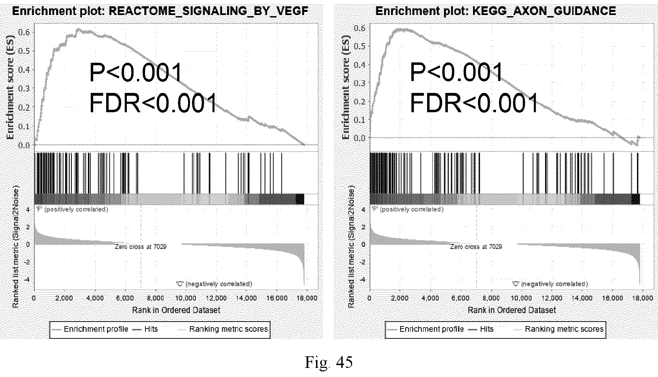

Six 6-week-old ob/ob obese (Jackson Lab B6.Cg-Lepob/J, Stock No: 000632) male mice from Jackson Laboratory with similar physical status were selected. Each mouse was fed a high-fat, high-sugar diet (HFSD, D11092103; Research Diet Inc) daily for 45 days to mimic fatty liver (NASH) (Kristiansen et al., 2016; doi: 10.4254/wjh.v8.i16.673). After 45 days, the mice were fed a high-fat, high-sugar diet continuously and were divided into two groups. The number of mice in each group was 3. For the experimental group, 30 mg/kg of therapeutic agent A and 5 mg/kg of therapeutic agent B were effectively administered to each mouse per day; for the control group, only the same amount of PBS solvent was administered to each mouse every day as a blank control. The experiment was conducted 7 days in total. After the experiment, skeletal muscle and liver samples were collected, RNAseq transcriptomic sequencing analysis and GSEA (Gene Set Enrichment Analysis) analysis were performed, and the most significant gene tags and markers were taken out, as shown in Fig. 45.

Fig. 45 shows the results of transcriptomic sequencing analysis in NASH mice. It can be seen that, compared with the NASH control group (C), the pharmaceutical composition (F) of the present invention can effectively up-regulate the VEGF signaling pathway in the skeletal muscle and liver (P<0.001, FDR<0.001), bone muscle axon guidance (neuronal guidance, P<0.001, FDR<0.001), Ephrin signaling pathway (P<0.001, FDR=0.0068), and liver IGF (insulin-like) signaling pathway (P=0.0039, FDR=0.07) in the NASH mice within 7 days. Therefore, it can be considered that the pharmaceutical therapeutic agent A+B provided by the present invention can effectively promote angiogenesis, neuromuscular tissue, and insulin sensitivity, just like imitating exercise. (Hoier & Hellsten 2014 doi: 10.1111/micc.12117; Stark et al. 2015 doi: 10.1083/jcb.201502036; Lavin et al. 2020 doi: 10.3389/fphys.2020.00653; Sarvas et al. 2015 doi: 10.14814/phy2.12277).

### Example 46

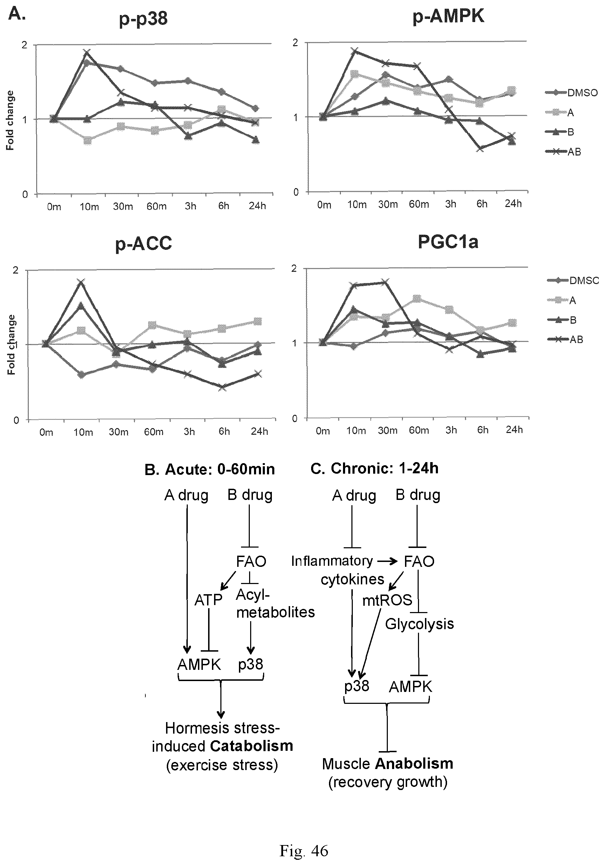

In vitro human primary skeletal muscle cells were used in the test, the cells were divided into four groups, and each group had six time points. For the experimental group, 100 mg/L of therapeutic agent A and 2 mg/L of therapeutic agent B were administered; for the control group 1, 100 mg/L of therapeutic agent A was administered; for the control group 2, 2 mg/L of therapeutic agent B was administered; and for the control group 3, the same amount of DMSO solvent was used as a blank control. The experiment was conducted 24 hours in total. The samples at each time point were then subjected to protein Western blot analysis, as shown in Fig. 46.

Fig. 46 shows the results of protein Western blot analysis of primary human skeletal muscle cells in vitro.

Fig. 46A shows that after the short-term administration (within 60 minutes), compared to control groups 1 to 3, in the experimental group using the pharmaceutical composition according to some embodiments of the present invention, the pharmaceutical therapeutic agents A+B can rapidly and significantly upregulate phospho-p38, phospho-AMPK, phospho-ACC and PGC1a. Compared with the control group 3, the control group 1 and control group 2 could also up-regulate phospho-p38, phospho-AMPK, phospho-ACC and PGC1a with treatment A or treatment B alone, but their effects were weaker or slower, and the time was not uniform. It can be seen that, after the short-term administration, the pharmaceutical therapeutic agents A+B provided by the present invention can more effectively promote the p38 and AMPK signaling pathways and downstream targets thereof than the single use of the therapeutic agent A or the single use of the therapeutic agent B, thereby promoting catabolism such as fat hydrolysis and fatty acid oxidation. After the long-term administration (3 to 24 hours the pharmaceutical therapeutic agents A+B can significantly down-regulate phospho-p38, phospho-AMPK, phospho-ACC and PGC1a in the experimental group using the pharmaceutical composition according to some embodiments of the present invention as compared with the control groups 1 to 3. Compared with the control group 3, the control group 1 and control group 2 could also down-regulate phospho-p38, phospho-ACC and PGC1a within 24 hours with either treatment A or treatment B alone, but the effect was weaker or slower, and the time was not uniform. It can be seen that after the long-term administration, the pharmaceutical therapeutic agents A+B provided by the present invention can more effectively inhibit the p38 and AMPK signaling pathways and downstream targets thereof than the therapeutic agent A or the therapeutic agent B alone, thereby promoting the anabolism.

Fig. 46B shows that after short-term administration (within 60 minutes), the pharmaceutical therapeutic agents A+B provided by the present invention can jointly regulate the mechanism model of p38 and AMPK signaling pathways, through fatty acid oxidation (FAO), fatty acid metabolites (acyl-metabolites), and adenosine triphosphate (ATP). It can be seen that the pharmaceutical therapeutic agents A+B can simultaneously enhance the p38 and AMPK signaling pathways after short-term administration, thereby promoting catabolism such as fat hydrolysis and fatty acid oxidation.

Fig. 46C shows that after long-term administration (3 to 24 hours), the pharmaceutical therapeutic agents A+B provided by the present invention can jointly regulate the mechanism model of p38 and AMPK signaling pathways, through inflammatory cytokines (Inf cytokines), fatty acid oxidation (FAO), mitochondrial reactive oxygen species (mtROS), and glycolysis. Thus, the pharmaceutical therapeutic agents A+B can cause a simultaneous sharp drop in p38 and AMPK signaling pathways after long-term administration, thereby promoting anabolism and muscle repair.

Therefore, daily administration of the pharmaceutical therapeutic agents A+B can specifically cycle the technetium p38 and AMPK signaling pathways and related metabolic changes, forming a cycle of excitatory effects, just like imitating exercise.

### Example 47

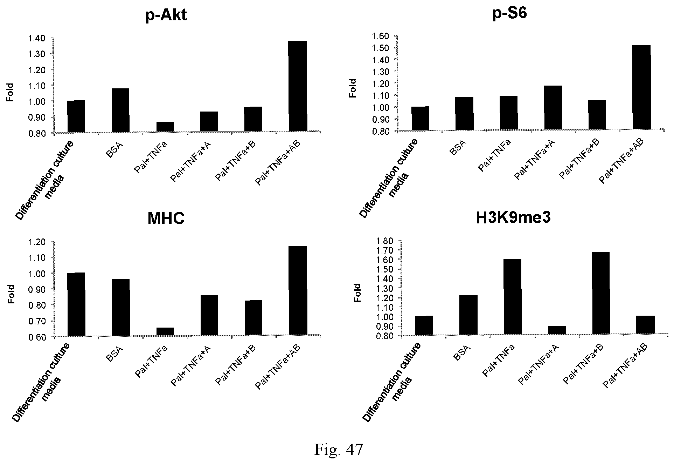

In vitro human primary skeletal muscle cells were used in the experiment, the cells were divided into six groups: for control group 1, the same amount of DMSO solvent was administered for 7 days as a blank control; for control group 2, the same amount of bovine serum albumin (BSA) was administered for 7 days as a fat solvent control; for control group 3, palmitic acid (Pal) and TNFα were administered for 7 days as a high-fat and inflammation-induced insulin resistance control; for control group 4, palmitic acid (Pal) and TNFα were administered for 7 days and 100 mg/L of therapeutic agent A was administered on day 4; for control group 5, palmitic acid (Pal) and TNFα were administered for 7 days and 2 mg/L of therapeutic agent B was administered on day 4; for the experimental group, palmitic acid (Pal) and TNFα were administered for 7 days and 100 mg/L of therapeutic agent A and 2 mg/L of therapeutic agent B were administered on day 4; afterwards, protein Western blot analysis was performed on each group of samples, as shown in Fig. 47.

Fig. 47 shows the results of protein Western blot analysis of primary human skeletal muscle cells induced insulin resistance in vitro 7 days post administration. After the 7-day test, in the experimental group treated with the pharmaceutical composition according to some embodiments of the present invention, the pharmaceutical therapeutic agents A+B can more significantly up-regulate the insulin sensitivity indicators phospho-Akt, phospho- S6 and myosin heavy chain (MHC) and down-regulate cellular senescence indicator H3K9me3, and restore to the level of control groups 1 and 2. Compared with the control group 3, the control group 4 and control group 5 can also up-regulate insulin sensitivity indicators phospho-Akt, phospho-S6 and myosin heavy chain by treating with the therapeutic agent A or the therapeutic agent B alone, but the effects are comparably weak. Compared with control group 3, the therapeutic agent A alone in the control group 4 can down-regulate the cellular senescence indicator H3K9me3 and restore it to the level of the control group 1, but therapeutic agent B alone in the control group 5 cannot achieve the same effect. It can be seen that the pharmaceutical therapeutic agents A+B provided by the present invention can more effectively promote insulin sensitivity and reverse cell senescence under the condition of insulin resistance than the therapeutic agent A or the therapeutic agent B alone.

### Example 48

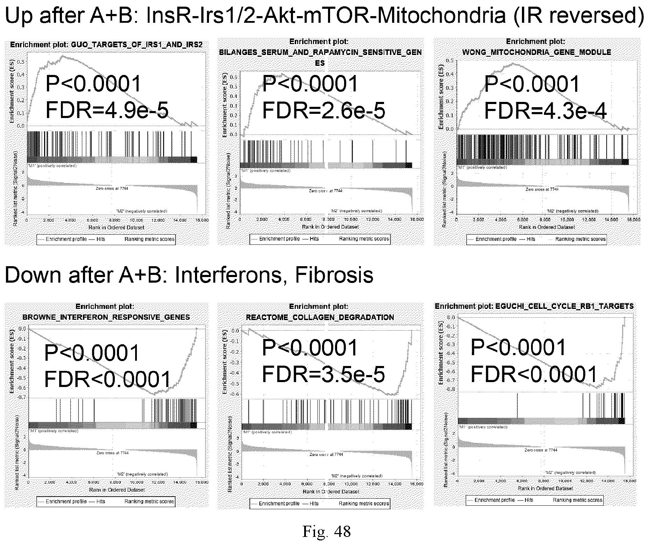

Similarly, skeletal muscle RNAseq transcriptomic sequencing analysis and GSEA (Gene Set Enrichment Analysis) analysis were also performed on each group of rats in Example 20, and the most significant gene tags and markers were taken out, as shown in Fig. 48.

Fig. 48 shows the results of skeletal muscle transcriptomic sequencing analysis induced by high-fat and high-sugar diet in PCOS polycystic ovary syndrome rats. It can be seen that compared with the PCOS rat control group (M2), IRS1 and IRS2 insulin receptor pathway targets (P<0.0001, FDR=4.9e-5), Rapamycin-sensitive PI3K-Akt-mTOR pathway targets (P<0.0001, FDR=2.6e-5), and mitochondria mitochondrial genes in the rats in the A+B experimental group (M1) were significantly increased. It can also be seen that, compared with the control group (M2) of the rats with polycystic ovary syndrome, inflammation-related interferon target (P<0.0001, FDR<0.0001), fibrosis-related collagen pathway target (P<0.0001, FDR=3.5e-5) and mesenchymal cell division target (P<0.0001, FDR<0.0001) in the rats in the A+B experimental group (M1) were significantly decreased. Therefore, it can be seen that the polycystic ovary syndrome rats treated with the pharmaceutical composition of the present invention can restore normal insulin (insulin/IGF-IRS-PI3K-mTOR) sensitivity and mitochondrial metabolism, and alleviate inflammation and fibrosis, thereby reversing insulin resistance, sarcopenia syndrome and metabolic syndrome.

### Example 49

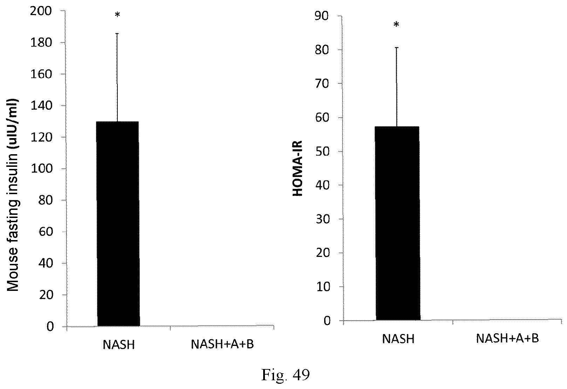

Similarly, fasting insulin enzyme-linked immunosorbent assay (ELISA, Abcam) was performed on the serum samples of the mice in each group of Example 33, and the insulin resistance indicator HOMA-IR was calculated, as shown in Fig. 49.

Fig. 49 shows insulin ELISA results and insulin resistance indicator HOMA-IR results in serum samples in NASH mice. It can be seen that, compared with the NASH control group, the pharmaceutical composition A+B of the present invention can effectively reduce serum fasting insulin (P<0.05) and insulin resistance indicator (P<0.05). Therefore, it can be considered that the pharmaceutical therapeutic agents A+B provided by the present invention can effectively promote the insulin sensitivity of NASH patients and relieve insulin resistance and hyperinsulinemia, just like imitating exercise (van der Windt et al. 2018, doi: 10.3727/ 105221617X15124844266408).

### Example 50

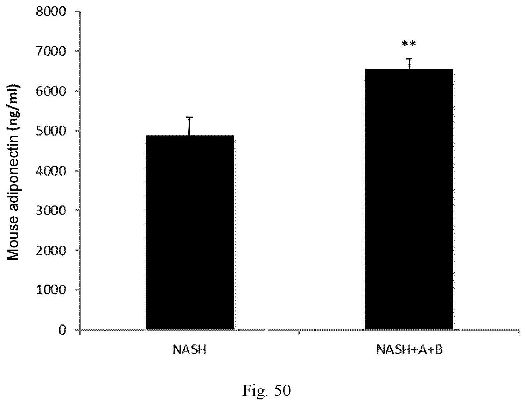

Similarly, adiponectin enzyme-linked immunosorbent assay (ELISA, Abcam) was performed on the serum samples of the mice in each group of Example 33, as shown in Fig. 50.

Fig. 50 shows adiponectin ELISA results in serum samples in NASH mice. It can be seen that, compared with the NASH control group, the pharmaceutical composition A+B of the present invention can effectively up-regulate serum adiponectin (P<0.001). Therefore, it can be considered that the pharmaceutical therapeutic agent A+B provided by the present invention can effectively promote glucose and lipid metabolism and inhibit inflammation through adiponectin, just like imitating exercise (Simpson & Singh 2008, doi: 10.1038/oby.2007.53).

### Example 51

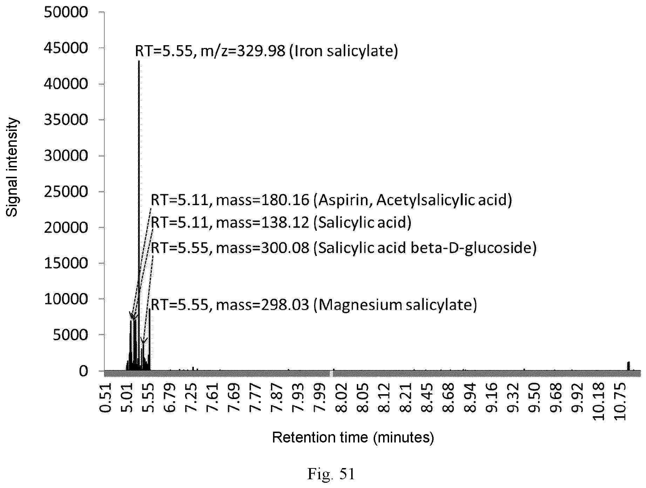

Mice were grouped and administered according to the same method as in Example 8, except that serum samples from each group of mice were subjected to liquid phase mass spectrometry (LC-MS, Waters^{™} XBridge C18 column and Xevo G2-XS) analysis 1 hour later, and the most prominent small molecule markers were taken out, as shown in Fig. 51.

Fig. 51 shows the comparative results of liquid mass spectrometry analysis in serum samples of mice in control group 1 and control group 3. It can be seen that compared to control group 3 (blank control), drug A (control group 1) can effectively form a variety of salicylate derivatives in serum, and most of the retention times are between 5 and 5.5 minutes, similar to that of acetylsalicylic acid (https://mona.fiehnlab.ucdavis.edu/spectra/display/EQ357853). Therefore, it can be considered that any salicylic acid derivative can imitate the efficacy of drug A in an obese body.

In light of the above examples, it can be easily seen that the pharmaceutical composition according to the present invention exhibits a very good synergistic effect in blood sugar control as compared to the use of individual components in the pharmaceutical composition of the present invention alone, especially in terms of fasting plasma glucose and postprandial plasma glucose reduction. At the same time, the present invention can effectively treat or prevent metabolic syndrome diseases caused by obesity, fatty liver, type 2 diabetes and insulin resistance.

## Claims

1. A combination of salicylate, or a pharmaceutically acceptable salt thereof, and trimetazidine, or a pharmaceutically acceptable salt thereof for use in treating obesity, non-alcoholic fatty liver, non-alcoholic steatohepatitis (NASH), metabolic syndrome, atherosclerosis, dyslipidemia, hyperlipidemia, or polycystic ovary syndrome (PCOS) in a subject in need thereof.

2. The combination for use according to claim 1, wherein the subject is diagnosed with one or more conditions selected from the group consisting of NASH, PCOS, and obesity; optionally wherein the obesity comprises visceral obesity, and/or abdominal obesity.

3. The combination for use according to claim 1 or 2, wherein the salicylate or pharmaceutically acceptable salt thereof is aspirin or a pharmaceutically acceptable salt thereof.

4. The combination for use according to any one of claims 1 to 3, wherein a weight ratio of the salicylate or pharmaceutically acceptable salt thereof to the trimetazidine or pharmaceutically acceptable salt thereof is from 1:1 to 10:1, optionally 6:1.

5. The combination for use according to any one of claims 1 to 4, wherein the salicylate or pharmaceutically acceptable salt thereof and the trimetazidine or pharmaceutically acceptable salt thereof are administered simultaneously, optionally wherein the salicylate or pharmaceutically acceptable salt thereof and the trimetazidine or pharmaceutically acceptable salt thereof are contained in a single dosage form.

6. The combination for use according to any one of claims 1 to 4, wherein the salicylate or pharmaceutically acceptable salt thereof and the trimetazidine or pharmaceutically acceptable salt thereof are administered separately.

7. The combination for use according to claim 6, wherein the salicylate or pharmaceutically acceptable salt thereof is administered before the trimetazidine or pharmaceutically acceptable salt thereof.

8. The combination for use according to claim 6, wherein the salicylate or pharmaceutically acceptable salt thereof is administered after the trimetazidine or pharmaceutically acceptable salt thereof.

9. The combination for use according to any one of claims 1 to 8, wherein the salicylate or pharmaceutically acceptable salt thereof and the trimetazidine or pharmaceutically acceptable salt thereof are administered orally or by injection.

10. The combination for use according to any one of claims 1 to 9, wherein the subject is a human.

11. The combination for use according to any one of claims 1 to 10, wherein the salicylate or pharmaceutically acceptable salt thereof and the trimetazidine or pharmaceutically acceptable salt thereof are for administration one or more times per day, optionally once per day.

## Patentansprüche

1. Kombination von Salicylat oder einem pharmazeutisch unbedenklichen Salz davon und Trimetazidin oder einem pharmazeutisch unbedenklichen Salz davon zur Verwendung beim Behandeln von Adipositas, nicht-alkoholischer Fettlebererkrankung, nicht-alkoholischer Steatohepatitis (NASH), metabolischem Syndrom, Atherosklerose, Dyslipidämie, Hyperlipidämie oder polyzystischem Ovarialsyndrom (PCOS) in einem dies bedürfenden Subjekt.

2. Kombination zur Verwendung nach Anspruch 1, wobei bei dem Subjekt eine oder mehrere der Erkrankungen, die ausgewählt sind aus der Gruppe bestehend aus NASH, PCOS und Adipositas, diagnostiziert wurden; wobei die Adipositas optional viszerale Adipositas und/oder abdominale Adipositas umfasst.

3. Kombination zur Verwendung nach Anspruch 1 oder 2, wobei das Salicylat oder das pharmazeutisch unbedenkliche Salz davon Aspirin oder ein pharmazeutisch unbedenkliches Salz davon ist.

4. Kombination zur Verwendung nach einem der Ansprüche 1 bis 3, wobei ein Gewichtsverhältnis des Salicylats oder des pharmazeutisch unbedenklichen Salzes davon zu dem Trimetazidin oder dem pharmazeutisch unbedenklichen Salz davon zwischen 1:1 und 10:1, optional bei 6:1 liegt.

5. Kombination zur Verwendung nach einem der Ansprüche 1 bis 4, wobei das Salicylat oder das pharmazeutisch unbedenkliche Salz davon und das Trimetazidin oder das pharmazeutisch unbedenkliche Salz davon gleichzeitig verabreicht werden, wobei das Salicylat oder das pharmazeutisch unbedenkliche Salz davon und das Trimetazidin oder das pharmazeutisch unbedenkliche Salz davon optional in einer einzigen Darreichungsform verabreicht werden.

6. Kombination zur Verwendung nach einem der Ansprüche 1 bis 4, wobei das Salicylat oder das pharmazeutisch unbedenkliche Salz davon und das Trimetazidin oder das pharmazeutisch unbedenkliche Salz davon getrennt verabreicht werden.

7. Kombination zur Verwendung nach Anspruch 6, wobei das Salicylat oder das pharmazeutisch unbedenkliche Salz davon vor dem Trimetazidin oder dem pharmazeutisch unbedenklichen Salz davon verabreicht wird.

8. Kombination zur Verwendung nach Anspruch 6, wobei das Salicylat oder das pharmazeutisch unbedenkliche Salz davon nach dem Trimetazidin oder dem pharmazeutisch unbedenklichen Salz davon verabreicht wird.

9. Kombination zur Verwendung nach einem der Ansprüche 1 bis 8, wobei das Salicylat oder das pharmazeutisch unbedenkliche Salz davon und das Trimetazidin oder das pharmazeutisch unbedenkliche Salz davon oral oder per Injektion verabreicht werden.

10. Kombination zur Verwendung nach einem der Ansprüche 1 bis 9, wobei das Subjekt ein Mensch ist.

11. Kombination zur Verwendung nach einem der Ansprüche 1 bis 10, wobei das Salicylat oder das pharmazeutisch unbedenkliche Salz davon und das Trimetazidin oder das pharmazeutisch unbedenkliche Salz davon zur Verabreichung einmal oder mehrmals pro Tag, optional einmal pro Tag, gedacht sind.

## Revendications

1. Combinaison de salicylate, ou d'un sel pharmaceutiquement acceptable de celui-ci, et de trimétazidine, ou d'un sel pharmaceutiquement acceptable de celle-ci, destinée à être utilisée dans le traitement de l'obésité, de la stéatose hépatique non alcoolique, de la stéato-hépatite non alcoolique (NASH), du syndrome métabolique, de l'athérosclérose, de la dyslipidémie, de l'hyperlipidémie ou du syndrome des ovaires polykystiques (SOPK) chez un sujet en ayant besoin.

2. Combinaison destinée à être utilisée selon la revendication 1, dans laquelle le sujet est diagnostiqué avec une ou plusieurs affections choisies dans le groupe constitué de la NASH, du SOPK et de l'obésité ; éventuellement dans lequel l'obésité comprend une obésité viscérale et/ou une obésité abdominale.

3. Combinaison destinée à être utilisée selon la revendication 1 ou 2, dans laquelle le salicylate ou un sel pharmaceutiquement acceptable de celui-ci est l'aspirine ou un sel pharmaceutiquement acceptable de celle-ci.

4. Combinaison destinée à être utilisée selon l'une quelconque des revendications 1 à 3, dans laquelle le rapport pondéral du salicylate ou d'un sel pharmaceutiquement acceptable de celui-ci à la trimétazidine ou à un sel pharmaceutiquement acceptable de celle-ci est de 1:1 à 10:1, éventuellement 6:1.

5. Combinaison destinée à être utilisée selon l'une quelconque des revendications 1 à 4, dans laquelle le salicylate ou un sel pharmaceutiquement acceptable de celui-ci et la trimétazidine ou un sel pharmaceutiquement acceptable de celle-ci sont administrés simultanément, éventuellement dans laquelle le salicylate ou un sel pharmaceutiquement acceptable de celui-ci et la trimétazidine ou un sel pharmaceutiquement acceptable de celle-ci sont contenus dans une forme posologique unique.

6. Combinaison destinée à être utilisée selon l'une quelconque des revendications 1 à 4, dans laquelle le salicylate ou un sel pharmaceutiquement acceptable de celui-ci et la trimétazidine ou un sel pharmaceutiquement acceptable de celle-ci sont administrés séparément.

7. Combinaison destinée à être utilisée selon la revendication 6, dans laquelle le salicylate ou un sel pharmaceutiquement acceptable de celui-ci est administré avant la trimétazidine ou un sel pharmaceutiquement acceptable de celle-ci.

8. Combinaison destinée à être utilisée selon la revendication 6, dans laquelle le salicylate ou un sel pharmaceutiquement acceptable de celui-ci est administré après la trimétazidine ou un sel pharmaceutiquement acceptable de celle-ci.

9. Combinaison destinée à être utilisée selon l'une quelconque des revendications 1 à 8, dans laquelle le salicylate ou un sel pharmaceutiquement acceptable de celui-ci et la trimétazidine ou un sel pharmaceutiquement acceptable de celle-ci sont administrés par voie orale ou par injection.

10. Combinaison destinée à être utilisée selon l'une quelconque des revendications 1 à 9, dans laquelle le sujet est un être humain.

11. Combinaison destinée à être utilisée selon l'une quelconque des revendications 1 à 10, dans laquelle le salicylate ou un sel pharmaceutiquement acceptable de celui-ci et la trimétazidine ou un sel pharmaceutiquement acceptable de celle-ci sont destinés à être administrés une ou plusieurs fois par jour, éventuellement une fois par jour.
